# EUROPEAN PATENT SPECIFICATION

(11) **EP 1 718 767 B1**
(45) Date of publication and mention of the grant of the patent: **11.04.2012**
(21) Application number: 05722705.0
(22) Date of filing: 03.02.2005
(51) Int. Cl.: A61K 39/395

(54) **COMPOSITIONS FOR TREATING BREAST AND PANCREATIC CANCER**
ZUSAMMENSETZUNGEN ZUR BEHANDLUNG VON BRUST- UND BAUCHSPEICHELDRÜSENKREBS
COMPOSITIONS PERMETTANT DE TRAITER DES CANCERS DU SEIN ET DU PANCREAS

(30) Priority: 03.02.2004 US 541527 P
(43) Date of publication of application: 08.11.2006
(60) Divisional application: 11152618.2
(73) Proprietor: THE REGENTS OF THE UNIVERSITY OF MICHIGAN, Ann Arbor, MI 48104-2592 (US)
(72) Inventor: CLARKE, Michael, F., Palo Alto, CA 94304 (US); AL-HAJJ, Muhammad, Cambridge, MA 02140 (US)
(74) Representative: Armitage, Ian Michael
(86) International application number: PCT/US2005/003419
(87) International publication number: WO 2005/074633

(56) References cited:
- WO-A-02/18544
- WO-A-03/050502
- WO-A2-00/20576
- US-A1- 2002 119 565
- US-A1- 2002 119 565
- US-A1- 2003 082 651
- US-B1- 6 379 925
- AL-HAJJ MUHAMMAD ET AL: "Prospective identification of tumorigenic breast cancer cells" PROCEEDINGS OF THE NATIONAL ACADEMY OF SCIENCES OF USA, NATIONAL ACADEMY OF SCIENCE, WASHINGTON, DC.; US, vol. 100, no. 7, 1 April 2003 (2003-04-01), pages 3983-3988, XP002470522 ISSN: 0027-8424
- WENG A P ET AL: "Growth suppression of pre-T acute lymphoblastic leukemia cells by inhibition of notch signaling" MOLECULAR AND CELLULAR BIOLOGY, AMERICAN SOCIETY FOR MICROBIOLOGY, WASHINGTON, US, vol. 23, no. 2, 1 January 2003 (2003-01-01), pages 655-664, XP003016923 ISSN: 0270-7306
- THELU J. ET AL: 'Notch signalling is linked to epidermal cell differentiation level in basal cell carcinoma,psoriasis and wound healing' BMC DERMATOLOGY vol. 2, no. 7, 2002, XP002299238
- CURRY C.L. ET AL: 'Gamma secretase inhibitor blocks Notch activation and induces apoptosis in Kaposi's sarcoma tumor cells' ONCOGENE vol. 24, 2005, pages 6333 - 6344, XP002997421
- HARPER J.A. ET AL: 'Notch signaling in development and disease' CLIN GENET. vol. 64, 2003, pages 461 - 472, XP002997422
- HOPFER O. ET AL: 'The Notch pathway in ovarian carcinomas and adenomas' BRITISH JOURNAL OF CANCER vol. 93, 2005, pages 709 - 718, XP002997423
- AXELSON H.: 'Notch signaling and cancer: emerging complexity' SEMINARS IN CANCER BIOLOGY vol. 14, 2004, pages 317 - 319, XP002997424
- POLITI K. ET AL: 'Notch in mammary gland development and breast cancer' SEMINARS IN CANCER BIOLOGY vol. 14, 2004, pages 341 - 347, XP002997425
- MAILLARD I. ET AL: 'Mastermind critically regulates Notch-mediated lymphoid cell fate decisions' BLOOD vol. 104, 2004, pages 1696 - 1702, XP002997426
- QIN J.-Z. ET AL: 'p53-independent NOXA induction overcomes apoptotic resistance of malignant melanomas' MOL CANCER THER vol. 3, 2004, pages 895 - 902, XP009058225
- HALLAHAN A.R. ET AL: 'The SmoA1 mouse model reveals that Notch signaling is critical for the growth and survival of sonic hedgehog-induced medulloblastomas' CANCER RESEARCH vol. 64, 2004, pages 7794 - 7800, XP002997427
- ALLENSPACH E.J. ET AL: 'Notch signaling in cancer' CANCER BIOLOGY & THERAPY vol. 1, September 2002 - October 2002, pages 466 - 476, XP008063765
- DONTU G. ET AL: 'Role of Notch signaling in cell-fate determination of human mammary stem/progenitor cells' BREAST CANCER RESEARCH vol. 6, no. 6, 2004, pages 605 - 615, XP002997428
- JANG M-S ET AL: "NOTCH SIGNALING AS A TARGET IN MULTIMODALITY CANCER THERAPY", CURRENT OPINION IN MOLECULAR THERAPEUTICS, CURRENT DRUGS, LONDON, GB, vol. 2, no. 1, 1 February 2000 (2000-02-01), pages 55-65, XP009013844, ISSN: 1464-8431
- PÈLEGRIN A ET AL: "[Immunotargeting of tumors: state of the art and prospects in 2000].", BULLETIN DU CANCER NOV 2000 LNKD- PUBMED:11125287, vol. 87, no. 11, November 2000 (2000-11), pages 777-791, ISSN: 0007-4551
- WENG ANDREW P ET AL: "Multiple niches for Notch in cancer: context is everything.", CURRENT OPINION IN GENETICS & DEVELOPMENT FEB 2004 LNKD- PUBMED:15108805, vol. 14, no. 1, February 2004 (2004-02), pages 48-54, ISSN: 0959-437X

## Description

### FIELD OF THE INVENTION

The present invention relates to compositions for treating cancer. The present invention provides compositions for inhibiting tumorigenesis of certain classes of cancer cells, including breast cancer cells and preventing metastasis.

### BACKGROUND

Cancer is one of the leading causes of death and metastatic cancer is often incurable⁶. Although current therapies can produce tumor regression, they rarely cure common tumors such as metastatic breast cancer⁶. Solid tumors consist of heterogeneous populations of cancer cells. Like acute myeloid leukemia (AML)⁷, it has been demonstrated recently that in most malignant human breast tumors, a small, distinct population of cancer cells are enriched for the ability to form tumors in immunodeficient mice¹. Previously it was shown that in 8 of the 9 tumors studied, the CD44⁺CD24^{-/low}Lineage⁻ population had the ability to form tumors when injected into immunodeficient mice. As few as 200 of these cells, termed "tumorigenic" cells, consistently formed tumors in mice. In contrast, the maj ority of the cancer cells in a tumor consisted of "non-tumorigenic" cells with alternative phenotypes. These cells failed to form tumor in NOD/SCID mice even when as many as 10⁴ cells were injected¹. In some tumors further enrichment of the tumorigenic cells was possible by isolating the ESA⁺CD44⁺ CD24^{-/ow}Lineage population of cancer cells. The ability to prospectively isolate the tumorigenic cancer cells permits investigation of critical biological pathways that represent therapeutic targets in these cells. The art is in need of additional systems and methods for characterizing, regulating, diagnosing, and treating cancer.

WO02/1854 disclosed a method and reagents for epithelial barrier formation and treatment of malignant and benign skin disorders by modulating the Notch pathway.

WO03/050502 reported that human breast tumors contain heterogeneous cancer cells. Using a xenograft model it was shown that only a small minority of breast cancer cells had the capacity to form new tumors. The tumorigenic cells could be distinguished from non-tumorigenic cancer cells based on surface marker expression. Notch activation was reported to promote survival of the tumorigenic cells and a blocking antibody against Notch4 induced tumorigenic breast cancer cells to undergo apoptosis.

### DESCRIPTION OF FIGURES

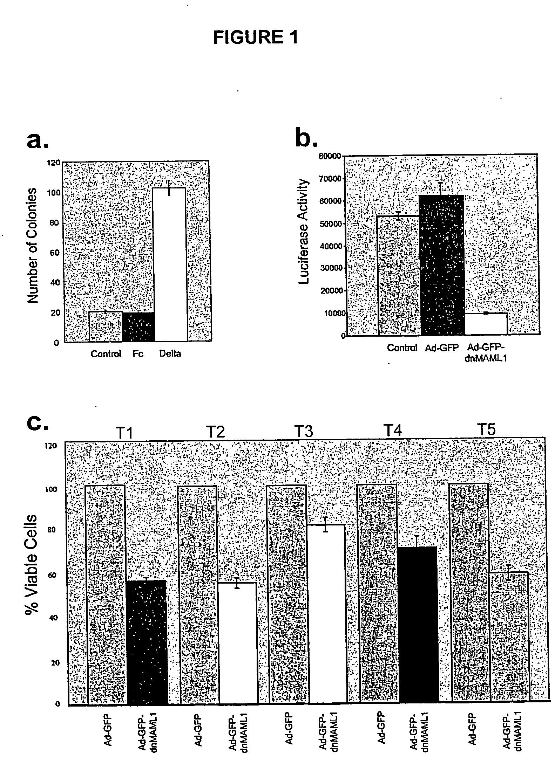

Figure 1 shows Notch signaling in breast cancer cells. Figure 1A shows that soluble Delta promotes colony formation. ESA⁺CD44⁺CD24^{-/low}Lineage T1 tumorigenic breast cancer cells were sorted and plated on collagen-coated plates in tissue culture medium alone (control), medium supplemented with the Fc control fragment¹¹ (Fc), or medium supplemented with Delta-Fc (Delta). Culture of cells with soluble Delta-Fc resulted in 5 fold more colonies than either the control cells or cells treated with just the control Fc fragment¹¹. Figure 1B shows inhibition of Notch signaling by a dominant-negative adenovirus vector. MCF-7 cells were stably transfected with a luciferase minigene under the regulation of the Notch-responsive HES-1 promoter³⁸. Luciferase activity was then measured in control MCF-7 cells cultured in medium containing Delta-Fc with no virus (Control) or 48 hours after infection with a GFP only adenovirus (Ad-GFP) or the dominant negative adenoviral vector dnMAML1 (Ad-GFP-dnMAML1). The Ad-GFP-dnMAML1 adenovirus, but not the Ad-GFP adenovirus, significantly decreased luciferase activity. Figure 1C shows that effect of Notch receptor transcriptional down-regulation on primary tumor cells. ESA⁺CD44⁺CD24^{-/low}Lineage cancer cells¹ (T1) or Lineage cancer cells from different patients' (T2-T5) were sorted in 12-well, collagen coated tissue culture plates. Cells were infected with the dominant negative adenoviral construct or the control GFP virus as indicated. Trypan blue exclusion was used to count viable cells 48 hours after infection. dnMAML1 significantly decreases the number of viable cells in T1, T2 T4and T5 while having a lesser effect on T3 cells.

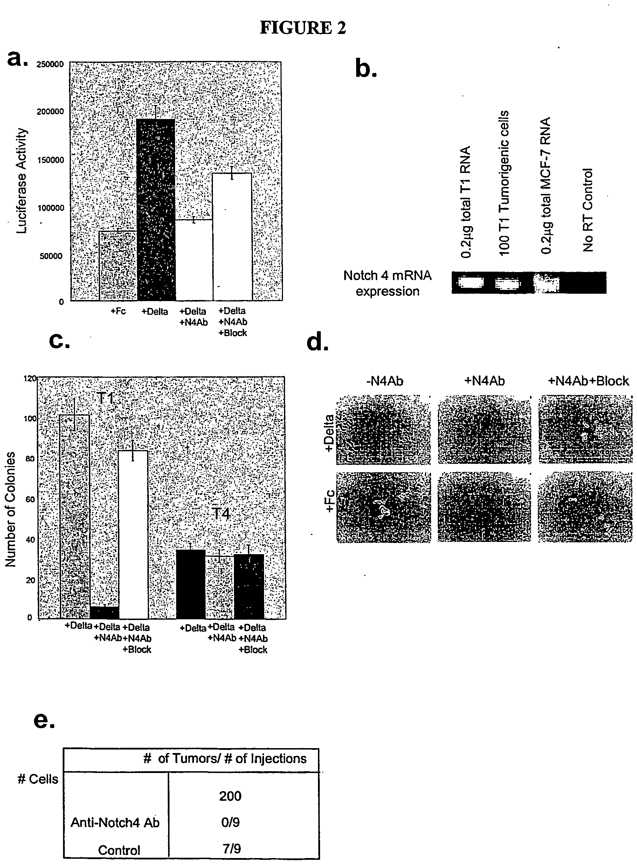

Figure 2 shows Notch4 signaling in tumorigenic breast cancer cells. Figure 2A shows results of a Notch pathway reporter gene assay. Luciferase activity was measured in extracts obtained from MCF-7 cells stably transfected with the HES-1/luciferase reporter gene that had been incubated with the control Fc fragment (Fc), soluble Delta-Fc (+Delta), soluble Delta-Fc and an anti-Notch4 polyclonal antibody (+N4Ab), or with soluble Delta-Fc, the anti-Notch4 polyclonal antibody as well as the blocking peptide against which the polyclonal antibody was made (+Block). The anti-Notch4 antibody inhibited luciferase activity, and this inhibition was partially reversed by preincubation of the blocking antibody with the blocking peptide. Figure 2B shows Notch4 expression by breast cancer cells. Unsorted cancer cells and tumorigenic (CD44⁺CD24^{-/low}Lineage⁻) T1 cells that had been passaged once in NOD/SCID mice were analyzed by RT-PCR for expression of Notch4. RT-PCR was also done to confirm that the MCF-7 cells that were used expressed Notch4 mRNA. Figure 2C shows colony formation. 20,000 T1 or T4 cancer cells were grown for 14 days in the indicated tissue culture medium containing either Delta-Fc (+Delta) or the Fc fragment alone (+Fc). Triplicate cultures were performed using the indicated medium supplemented with no antibody (+Delta), the polyclonal anti-Notch4 antibody (+N4Ab), or the anti-Notch4 antibody plus blocking peptide (+N4Ab + Block). Figure 2D shows representative pictures of colonies that formed after 14 days in each condition are shown (10x objective), and the table indicates the number of colonies counted in each well. Soluble Delta-Fc stimulated colony formation (p<0. 001), while the polyclonal anti-Notch4 antibody inhibited colony formation in the presence of Delta-Fc (p<0.001). When the antibody was pre-incubated with the peptide used to generate the anti-Notch4 antibody, the inhibitory effect of the antibody was nearly completely reversed (p<0.001). Results are representative of 3 independent experiments. Figure 2E shows ESA⁺CD44⁺CD24^{-/low}Lineage⁻ tumorigenic cells were isolated from first or second passage T1 tumor. 200 cells were injected into the area of the mammary fat pads of mice in control buffer or after being incubated with a polyclonal anti-Notch4 antibody. Tumor formation was monitored over a five-month period. The results are from 2 independent experiments.

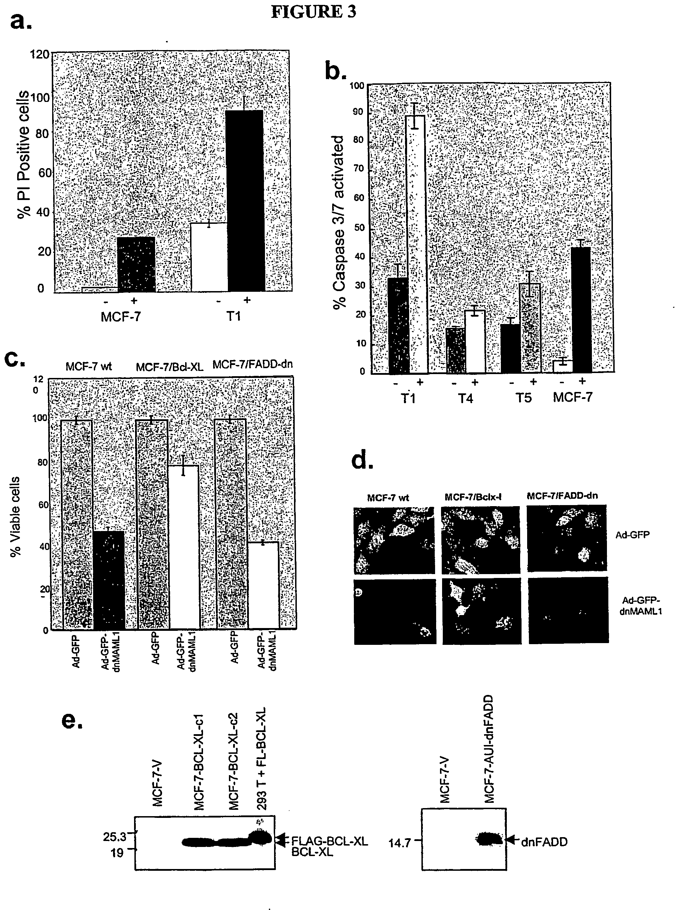

Figure 3 shows a mechanism of apoptosis. Figure 3A shows viability of cancer cells treated with the anti-Notch4 antibody. ESA⁺CD44⁺CD24^{-/low}Lineage⁻ tumorigenic Tumor 1 (T1) cells or MCF-7 cells were cultured with the anti-Notch4 antibody for 48 hours. Cells were harvested and stained with PI and viability was measured by flow cytometry. Figure 3B shows caspase activation by the anti-Notch4 antibody. ESA⁺CD44⁺CD24^{-/low}Lineage tumorigenic Tumor 1 (T1) cells, H2K⁻ Tumor 4 (T4) or Tumor 5 (T5) cells, or MCF-7 cells were cultured in control medium (-) or medium with the anti-Notch4 antibody (+), and 36 hours later active caspase 3/7 was measured by flow cytometry using a fluorescent substrate CaspoTag^{™}. After exposure to the anti-Notch4 antibody, the percentage of cells expressing activated caspase 3/7 was markedly increased in T1 tumor-initiating cells, T5 cells and MCF-7 cells as compared to control cells. T4 cells did not exhibit the high level of caspase activation. Figure 3C and 3D show the effect of inhibition of Notch transcriptional transactivation on MCF-7 cells. Wild type MCF-7 cells, MCF-7 cells that stably transfected with Bcl.-X_{L}, or a dominant-negative FADD (dnFADD) were infected with the dnMAML1 or a control GFP adenovirus. After 48 hours, the percentage of viable cells that had been infected with the dnMAML1 AD relative to cells infected with the control adenovirus is shown Figure 3C. Figure 3D shows a photomicrograph (20x objective) of each cell type infected with either the control GFP or GFP-dnMAML1 adenovirus as indicated. Figure 3E shows the expression level of dnFADD and BCL-X_{L} in the MCF-7 cell lines. Western blots were done to determine the expression of the indicated protein by the parental MCF-7 cells (MCF-7wt), MCF-7 cells transfected with minigenes expressing the dominant-negative FADD (MCF-7dnFADD) or two different clones transfected with BCL-X_{L} (MCF-7-BCL-X_{L}-c1 and MCF-7-BCL-X_{L}-c2). 293T cells transfected with FLAG-tagged BCL-X_{L} serve as a positive control for expression of BCL-X_{L}. dnMAML1 significantly decreases the number of viable cells in the parental MCF-7 cell line as well as the MCF-7/dnFADD cell line, while having only a minimal effect on the two MCF-7/Bcl-X_{L} cell lines.

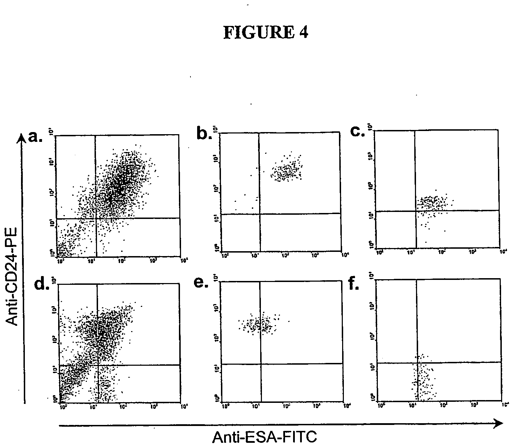

Figure 4 shows the isolation of tumorigenic and.nontumorigenic cancer cells. Flow cytometry was used to isolate subpopulations of Tumor 1 (a,b,c) or Tumor 4 (d,e,f) that had previously been tested for tumorigenicity in NOD/SCID mice¹. Cells were stained with antibodies against ESA, CD44, CD24, Lineage markers, mouse-H2K (for passaged tumors obtained from mice), and 7AAD. Deadcells (7AAD⁺), mouse cells (H2K⁺) and normal cells were eliminated from all analyses. Each plot depicts the ESA CD24 staining patterns of live human Lineage- cancer cells¹. The re-analyses of the sorted tumorigenic (c,f) and non-tumorigenic (b,e) populations of cells are shown. The tumorigenic cells were isolated based on detectable expression of CD44 (gates not shown). The ESA⁻CD24^{-/low}CD44⁺Lineage⁻ population of T4 cells had reduced, but present, tumorigenic potential¹.

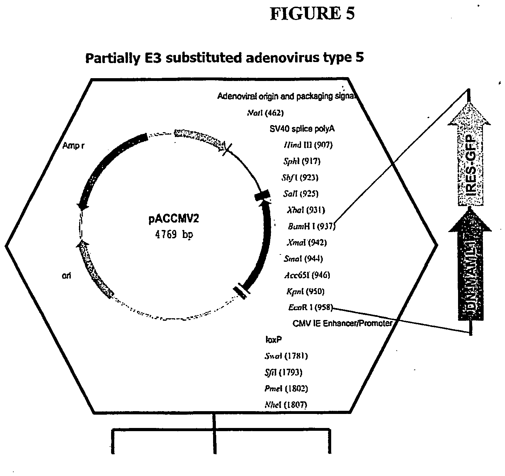

Figure 5 shows adenovirus inhibition of Notch signaling, showing depiction of the Ad-GFP-dnMAML1 viral construct. The coding region of amino acids 1-302 of the Human MAML1 was cloned into the MSCVneoEB vector upstream of the IRES-GFP gene. The dnMAML1-IRES-GFP fragment was then cloned into the adenovirus shuttle vector pACCMV2 at the EcoRI and BamHI sites.

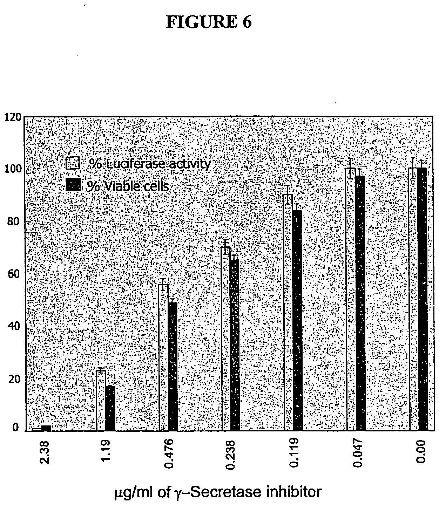

Figure 6 shows a graph demonstrating the effect of y-secretase inhibitor on MCF-7 cell viability and the Notch receptor activation. Cell viability was assayed by plating triplicate 20,000 MCF-7 cells that are shown to express Notch in 6-well plates. 48 hours later, varying amounts of y-secretase inhibitor were added to the cells. Total and Trypan Blue negative (viable) cells were counted 24 hours later and % cell viability was determined. Stably transfected MCF-7 cells with the reporter luciferase gene under the control of the HES-1 promoter were co-cultivated in 6-well plates with varying concentrations of the y-secretase inhibitor as above. The trans-activation or suppression of the HES1 promoter was monitored with a standard luciferase assay. The y-secretase inhibitor clearly suppresses the expression of the notch receptor and significantly decreases the cell's viability. These results demonstrate that y-secretase inhibitors find use in inhibition of Notch in tumorigenic cancer cells.

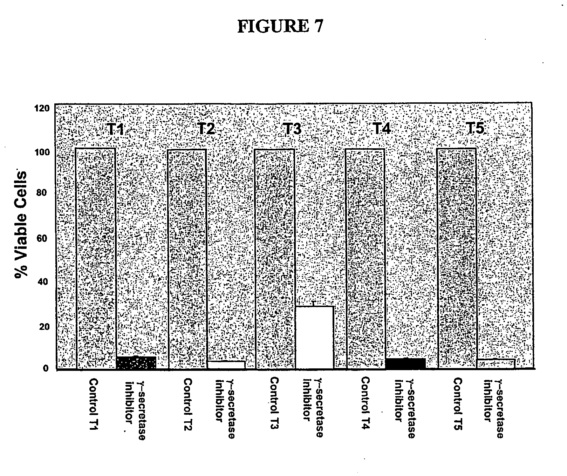

Figure 7 shows a graph of percent viable cells for cultured cancer cells treated with and without y-secretase inhibitors.

### DEFINITIONS

To facilitate an understanding of the present invention, a number of terms and phrases are defined below:

As used herein, the terms "anticancer agent," "conventional anticancer agent," or "cancer therapeutic drug" refer to any therapeutic agents (*e.g.*, chemotherapeutic compounds and/or molecular therapeutic compounds), radiation therapies, or surgical interventions, used in the treatment of cancer (*e.g.*, in mammals).

As used herein, the terms "drug" and "chemotherapeutic agent" refer to pharmacologically active molecules that are used to diagnose, treat, or prevent diseases or pathological conditions in a physiological system (*e.g.*, a subject, or *in vivo, in vitro,* or *ex vivo* cells, tissues, and organs). Drugs act by altering the physiology of a living organism, tissue, cell, or *in vitro* system to which the drug has been administered. It is intended that the terms "drug" and "chemotherapeutic agent" encompass anti-hyperproliferative and antineoplastic compounds as well as other biologically therapeutic compounds.

As used herein the term "prodrug" refers to a pharmacologically inactive derivative of a parent "drug" molecule that requires biotransformation (*e.g.*, either spontaneous or enzymatic) within the target physiological system to release, or to convert (*e.g.*, enzymatically, mechanically, electromagnetically, *etc.*) the "prodrug" into the active "drug." "Prodrugs" are designed to overcome problems associated with stability, toxicity, lack of specificity, or limited bioavailability. Exemplary "prodrugs" comprise an active "drug" molecule itself and a chemical masking group (*e.g.*, a group that reversibly suppresses the activity of the "drug"). Some preferred "prodrugs" are variations or derivatives of compounds that have groups cleavable under metabolic conditions. Exemplary "prodrugs" become pharmaceutically active *in vivo* or *in vitro* when they undergo solvolysis under physiological conditions or undergo enzymatic degradation or other biochemical transformation (*e.g.*, phosphorylation, hydrogenation, dehydrogenation, glycosylation, *etc*.). Prodrugs often offer advantages of solubility, tissue compatibility, or delayed release in the mammalian organism. (*See e.g*., Bundgard, Design of Prodrugs, pp. 7-9, 21-24, Elsevier, Amsterdam (1985); and Silverman, The Organic Chemistry of Drug Design and Drug Action, pp. 352-401, Academic Press, San Diego, CA (1992)). Common "prodrugs" include acid derivatives such as esters prepared by reaction of parent acids with a suitable alcohol (*e.g.*, a lower alkanol), amides prepared by reaction of the parent acid compound with an amine (*e.g.*, as described above), or basic groups reacted to form an acylated base derivative (*e.g.*, a lower alkylamide).

An "effective amount" is an amount sufficient to effect beneficial or desired results. An effective amount can be administered in one or more administrations.

As used herein, the term "administration" refers to the act of giving a drug, prodrug, antibody, or other agent, or therapeutic treatment to a physiological system (*e.g*., a subject or *in vivo*, *in vitro*, or *ex vivo* cells, tissues, and organs). Exemplary routes of administration to the human body can be through the eyes (opthalmic), mouth (oral), skin (transdermal), nose (nasal), lungs (inhalant), oral mucosa (buccal), ear, by injection (*e.g.*, intravenously, subcutaneously, intratumorally, intraperitoneally, *etc*.) and the like.

"Coadministration" refers to administration of more than one chemical agent or therapeutic treatment (*e.g.*, radiation therapy) to a physiological system (*e.g*., a subject or *in vivo, in vitro,* or *ex vivo* cells, tissues, and organs). "Coadministration" of the respective chemical agents and therapeutic treatments (*e.g.*, radiation therapy) may be concurrent, or in any temporal order or physical combination.

As used herein, the term "bioavailability" refers to any measure of the ability of an agent to be absorbed into a biological target fluid (*e.g.*, blood, cytoplasm, CNS fluid, and the like), tissue, organelle or intercellular space after administration to a physiological system (*e.g.*, a subject or *in vivo, in vitro,* or *ex vivo* cells, tissues, and organs).

As used herein, the term "biodistribution" refers to the location of an agent in organelles, cells (*e.g*., *in vivo* or *in vitro*), tissues, organs, or organisms, after administration to a physiological system.

A "hyperproliferative disease," as used herein refers to any condition in which a localized population of proliferating cells in an animal is not governed by the usual limitations of normal growth. Examples of hyperproliferative disorders include tumors, neoplasms, lymphomas and the like. A neoplasm is said to be benign if it does not undergo invasion or metastasis and malignant if it does either of these. A "metastatic" cell or tissue means that the cell can invade and destroy neighboring body structures. Hyperplasia is a form of cell proliferation involving an increase in cell number in a tissue or organ without significant alteration in structure or function. Metaplasia is a form of controlled cell growth in which one type of fully differentiated cell substitutes for another type of differentiated cell. Metaplasia can occur in epithelial or connective tissue cells. A typical metaplasia involves a somewhat disorderly metaplastic epithelium.

As used herein, the term "neoplastic disease" refers to any abnormal growth of cells or tissues being either benign (non-cancerous) or malignant (cancerous).

As used herein, the term "anti-neoplastic agent" refers to any compound that retards the proliferation, growth, or spread of a targeted (*e.g.*, malignant) neoplasm.

As used herein, the term "regression" refers to the return of a diseased subject, cell, tissue, or organ to a non-pathological, or less pathological state as compared to basal nonpathogenic exemplary subject, cell, tissue, or organ. For example, regression of a tumor includes a reduction of tumor mass as well as complete disappearance of a tumor or tumors.

As used herein, the terms "prevent," "preventing," and "prevention" refer to a decrease in the occurrence of hyperproliferative or neoplastic cells in a subject. The prevention may be complete, *e.g.*, the total absence of hyperproliferative or neoplastic cells in a subject. The prevention may also be partial, such that the occurrence of hyperproliferative or neoplastic cells in a subject is less than that which would have occurred without the present invention.

As used herein the term, "*in vitro*" refers to an artificial environment and to processes or reactions that occur within an artificial environment. *In vitro* environments can consist of, but are not limited to, test tubes and cell cultures. The term "*in vivo*" refers to the natural environment (*e.g.*, an animal or a cell) and to processes or reactions that occur within a natural environment.

As used herein, the term "cell culture" refers to any *in vitro* culture of cells. Included within this term are continuous cell lines (*e.g.*, with an immortal phenotype), primary cell cultures, finite cell lines (*e.g.*, non-transformed cells), and any other cell population maintained *in vitro,* including oocytes and embryos.

As used herein, the term "subject" refers to organisms to be treated by the methods of the present invention. Such organisms include, but are not limited to, humans and veterinary animals (dogs, cats, horses, pigs, cattle, sheep, goats, and the like). In the context of the invention, the term "subject" generally refers to an individual who will receive or who has received treatment.

The term "diagnosed," as used herein, refers to the recognition of a disease by its signs and symptoms or genetic analysis, pathological analysis, histological analysis, and the like.

As used herein, the term "competes for binding" is used in reference to a first molecule with an activity that binds to the same target as does a second molecule. The efficiency (*e.g.*, kinetics or thermodynamics) of binding by the first molecule may be the same as, or greater than, or less than, the efficiency of the target binding by the second molecule. For example, the equilibrium binding constant (Kd) for binding to the target may be different for the two molecules.

As used herein, the term "antisense" is used in reference to nucleic acid sequences (*e.g.*, RNA, phosphorothioate DNA) that are complementary to a specific RNA sequence (*e.g.*, mRNA). Included within this definition are natural or synthetic antisense RNA molecules, including molecules that regulate gene expression, such as small interfering RNAs or micro RNAs.

The term "test compound" refers to any chemical entity, pharmaceutical, drug, and the like, that can be used to treat or prevent a disease, illness, sickness, or disorder of bodily function, or otherwise alter the physiological or cellular status of a sample. Test compounds comprise both known and potential therapeutic compounds. A test compound can be determined to be therapeutic by using the screening methods disclosed herein. A "known therapeutic compound" refers to a therapeutic compound that has been shown (*e.g.*, through animal trials or prior experience with administration to humans) to be effective in such treatment or prevention. "Test compounds" may be anticancer agents, particularly anticancer agents that induce apoptosis in cells.

As used herein, the term "purified" or "to purify" refers to the removal of undesired components from a sample. As used herein, the term "substantially purified" refers to molecules (*e.g.*, polynucleotides, polypeptides, chemical compounds) that are removed from their natural environment, isolated or separated, and are at least 60% free, preferably at least 75% free, and most preferably at least 90% free from other components with which they are naturally associated. For example, an "isolated polynucleotide" is therefore a substantially purified polynucleotide.

"Nucleic acid sequence" and "nucleotide sequence" as used herein refer to an oligonucleotide or polynucleotide, and fragments or portions thereof, and to DNA or RNA of genomic or synthetic origin which may be single- or double-stranded, and represent the sense or antisense strand. As used herein, the terms "nucleic acid molecule encoding," "DNA sequence encoding," "DNA encoding," "RNA sequence encoding," and "RNA encoding" refer to the order or sequence of deoxyribonucleotides or ribonucleotides along a strand of deoxyribonucleic acid or ribonucleic acid. The order of these deoxyribonucleotides or ribonucleotides determines the order of amino acids along the polypeptide (protein) chain translated from the mRNA. The DNA or RNA sequence thus codes for the amino acid sequence.

The term "gene" refers to a nucleic acid (*e.g.*, DNA or RNA) sequence that comprises coding sequences necessary for the production of a polypeptide or precursor (*e.g.*, proinsulin). The polypeptide can be encoded by a full length coding sequence or by any portion of the coding sequence so long as the desired activity or functional properties (*e.g.*, enzymatic activity, ligand binding, signal transduction, *etc.*) of the full-length or fragment are retained. The term also encompasses the coding region of a structural gene and includes sequences located adjacent to the coding region on both the 5' and 3' ends for a distance of about 1 kb or more on either end such that the gene corresponds to the length of the full-length mRNA. The sequences that are located 5' of the coding region and which are present on the mRNA are referred to as 5' untranslated sequences. The sequences that are located 3' or downstream of the coding region and which are present on the mRNA are referred to as 3' untranslated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene which are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "exogenous gene" refers to a gene that is not naturally present in a host organism or cell, or is artificially introduced into a host organism or cell.

As used herein, the term "vector" refers to any genetic element, such as a plasmid, phage, transposon, cosmid, chromosome, virus, virion, *etc.*, which is capable of replication when associated with the proper control elements and which can transfer gene sequences between cells. Thus, the term includes cloning and expression vehicles, as well as viral vectors.

As used herein, the term "gene expression" refers to the process of converting genetic information encoded in a gene into RNA (*e.g.*, mRNA, rRNA, tRNA, or snRNA) through "transcription" of the gene (*i.e.,* via the enzymatic action of an RNA polymerase), and for protein encoding genes, into protein through "translation" of mRNA. Gene expression can be regulated at many stages in the process. "Up-regulation" or "activation" refers to regulation that increases the production of gene expression products (*i.e.*, RNA or protein), while "down-regulation" or "repression" refers to regulation that decreases production. Molecules (*e.g.*, transcription factors) that are involved in up-regulation or down-regulation are often called "activators" and "repressors," respectively.

As used herein, the term "antigen binding protein" refers to proteins which bind to a specific antigen. "Antigen binding proteins" include, but are not limited to, immunoglobulins, including polyclonal, monoclonal, chimeric, single chain, and humanized antibodies, Fab fragments, F(ab')2 fragments, and Fab expression libraries. Various procedures known in the art are used for the production of polyclonal antibodies. For the production of antibodies, various host animals can be immunized by injection with the peptide corresponding to the desired epitope including, but not limited to, rabbits, mice, rats, sheep, goats, *etc.* The peptide may be conjugated to an immunogenic carrier (*e.g.*, diphtheria toxoid, bovine serum albumin (BSA), or keyhole limpet hemocyanin (KLH)). Various adjuvants are used to increase the immunological response, depending on the host species, including, but not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and Corynebacterium parvum.

For preparation of monoclonal antibodies, any technique that provides for the production of antibody molecules by continuous cell lines in culture may be used (*See e.g.*, Harlow and Lane, Antibodies: A Laboratory Manual, Cold Spring Harbor Laboratory Press, Cold Spring Harbor, NY). These include, but are not limited to, the hybridoma technique originally developed by Köhler and Milstein (Köhler and Milstein, Nature, 256:495-497 (1975)), as well as the trioma technique, the human B-cell hybridoma technique (*See e.g.,* Kozbor et al., Immunol. Today, 4:72 (1983)), and the EBV-hybridoma technique to produce human monoclonal antibodies (Cole et al., in Monoclonal Antibodies and Cancer Therapy, Alan R. Liss, Inc., pp. 77-96 (1985)).

Techniques described for the production of single chain antibodies (U.S. 4,946,778) can be adapted to produce specific single chain antibodies as desired. The techniques known in the art for the construction of Fab expression libraries (Huse et al., Science, 246:1275-1281 (1989)) allow rapid and easy identification of monoclonal Fab fragments with the desired specificity.

Antibody fragments that contain the idiotype (antigen binding region) of the antibody molecule can be generated by known techniques. For example, such fragments include, but are not limited to: the F(ab')2 fragment that can be produced by pepsin digestion of an antibody molecule; the Fab' fragments that can be generated by reducing the disulfide bridges of an F(ab')2 fragment, and the Fab fragments that can be generated by treating an antibody molecule with papain and a reducing agent.

Genes encoding antigen-binding proteins can be isolated by methods known in the art. In the production of antibodies, screening for the desired antibody can be accomplished by techniques known in the art (*e.g.*, radioimmunoassay, ELISA (enzyme-linked immunosorbant assay), "sandwich" immunoassays, immunoradiometric assays, gel diffusion precipitin reactions, immunodiffusion assays, *in situ* immunoassays (using colloidal gold, enzyme or radioisotope labels, for example), Western Blots, precipitation reactions, agglutination assays (*e.g.*, gel agglutination assays, hemagglutination assays, *etc.*), complement fixation assays, immunofluorescence assays, protein A assays, and immunoelectrophoresis assays, *etc*.) *etc.*

As used herein, the term "modulate" refers to the activity of a compound to affect (*e.g*., to promote or retard) an aspect of the cellular function including, but not limited to, cell growth, proliferation, invasion, angiogenesis, apoptosis, and the like.

### SUMMARY OF THE INVENTION

The present invention provides an inhibitor of Notch signaling for use in a method of inhibiting tumorigeric cells to treat pancreatic cancer or breast cancer in a subject, wherein the inhibitor is an anti-Notch antibody that inhibits proliferation of tumorigeric cells, and wherein the method comprises co-administering the inhibitor and an anti-cancer agent to the subject, wherein the anticancer agent is a chemotherapeutic agent.

Also described herein are research and clinical diagnostic methods for identifying the presence of a tumorigenic cell in a tumor sample. Some methods comprise detecting one or more (e.g., 2, 3, 4, ...) markers or properties characteristic of the tumorigenic cell (e.g., one or more markers or properties that are not characteristic of a non-tumorigenic cell). The one or more markers may include, but are not limited to, expression of Notch4, cell death upon exposure to an anti-Notch4 antibody, expression of Manic Fringe, altered expression of a Notch ligand Delta relative to said non-tumorigenic cell, expression of Jagged, and cell death upon exposure to a dominant negative adenoviral vector dnMAML1. While the method is not limited by the nature of the cell, the cell may be from a breast cancer tumor.

The identification of the tumorigenic cell can be used in selecting a treatment course of action for a subject. For example, the treatment course of action may comprise administration of a Notch4 pathway inhibitor to the subject.

Also described herein are methods for selecting or characterizing compounds (e.g., for basic research, drug screening, drug trials, monitoring therapy, etc.), comprising the steps of: a) providing ample comprising a tumorigenic cell (e.g., breast cell); b) exposing the sample to a test compound; and c) detecting a change in the cell in response to the test compound. The test compound may comprise an antibody (e.g., an antibody that regulates a Notch signaling pathway) or an anti-neoplastic compound. A second or additional compound may be co-administered (e.g., a known anti-neoplastic therapeutic compound). The detecting step may comprise detecting cell death of said tumorigenic cell (e.g., detection of apoptosis markers such as caspace, etc.).

Also described herein are methods for identifying subjects having tumorigenic cells and treating the subject with an appropriate treatment course of action based on the nature of the identified tumorigenic cell. For example, a method for treating a subject having tumorigenic cells (e.g., breast cells), comprising the steps of: a) identifying the presence of a tumorigenic breast cell in the subject; b) identifying one or more markers or properties characteristic of the tumorigenic cell to identify the nature of the tumorigenic cell; and c) selecting a therapeutic course of action based on the nature of the tumorigenic cell. The course of action may comprise administration of a Notch 4 pathway inhibitor or other appropriate therapeutic to the subject when the tumorigenic cell is characterized as expressing Notch4, or for example, where the tumorigenic cell does not express Notch4, but where neighboring, non-tumorigenic cells express Notch 4. The course of action may comprise surgical removal of a tumor from the subject and administration of a Notch4 pathway inhibitor or other appropriate therapeutic compound to the subject (e.g., to prevent tumorigenesis or metastasis caused by remaining tumorigenic cells. The course of action may comprise co-administration of a Notch4 pathway inhibitor or other appropriate therapeutic compound and a second anti-neoplastic agent to the subject. As described herein, a Notch pathway inhibitor may be used in methods of preventing or reducing metastasis, comprising the step of administering the Notch pathway inhibitor to a subject suspected of having metastasis (e.g., suspected of undergoing metastasis or a risk of metastasis). The Notch pathway inhibitor may comprise an anti-Notch4 antibody. The administration may be conducted in conjunction with removal of a solid tumor (e.g., a breast tumor) from the subject.

### DETAINED DESCRIPTION OF THE INVENTION

Solid tumors consist of heterogeneous populations of cancer cells that differ in their ability to form new tumors. Cancer cells that have the ability to form tumors (i.e., tumorigenic cancer cells) and cancer cells that lack this capacity (i,e., non-tumorigenic cancer cells) can be distinguished based on phenotype¹. Since tumorigenic cancer cells and normal stem cells share the fundamental ability to replicate themselves through the process of self-renewal, experiments conducted during the development of the present invention investigated potential self-renewal pathways in tumorigenic cancer cells so as to allow for the characterization and identification of tumorigenic cancer cells, provide systems for screening cancer therapeutics, and provide therapeutic targets and compounds directed to those targets.

Thus, the present invention relates to compositions for use in methods of treating cancer, and provides compositions for inhibiting tumorigenesis of certain classes of cancer cells, including breast cancer cells.

Also disclosed are methods for identifying tumorigenic cells and diagnosing diseases (e.g., hyperproliferative diseases) or biological events (e.g., tumor metastasis) associated with the presence of tumorigenic cells. In particular, the present disclosure identifies classes of cells within cancers that are tumorigenic and provides detectable characteristics of such cells, such that their presence can be determined, for example, in choosing whether to submit a subject to a medical intervention, selecting an appropriate treatment course of action, monitoing the success or progress of a therapeutic course of action (e.g., in a drug trial or in selecting individualized, ongoing therapy), or screening for new therapeutic compounds or therapeutic targets.

The present disclosure also identifies biological targets and regulators of those biological targets that modulate tumorigenesis and metastasis. Therapeutic methods can be used, in combination with other therapeutic courses of action (e.g., coadministration of other anti-neoplastic agents) or with diagnostic procedures (e.g., patients that are amenable to the therapeutic methods of the present invention are identified by the diagnostic methods described herein).

Systems and methods are described for identifying the genes and proteins expressed by tumorigenic cells to identify proteins whose function is necessary for tumorigenesis, providing novel drug targets.

The expression of Notch proteins and/or regulators of the Notch signaling pathways can be used to identify tumorigenic cells. Regulators of Notch proteins and/or Notch signaling pathways also find use in research, drug screening, and therapeutic methods. For example, inhibitors of Notch expression or function (e.g., Notch4) find use in preventing or reducing cell proliferation, hyperproliferative disease development or progression, and cancer metastasis. Inhibitors can be utilized following removal of a solid tumor mass to help reduce proliferation and metastasis of remaining hyperproliferative cells. For example, it is shown herein that non-tumorigenic cells (e.g., cells that may be removed in the excision of a solid tumor mass) express factors that prevent neighboring cells from undergoing proliferation. Thus, appropriate therapeutic intervention following tumor removal provides a substitute for this function, suppressing proliferation and metastasis of remaining. tumorigenic cells.

The present invention relates to compositions for use in treating breast cancer or pancreatic cancer, as defined by the claims.

The Notch pathway is important for the maintenance of germ cells and a variety of normal stem cells^{2,3}, and Notch mutations lead to breast cancer in mice^{4,5}. The present invention demonstrates that in a subset of cancers (e.g., human breast cancers), the Notch agonist Delta provides a survival signal for tumorigenic breast cancer cells allowing them to form colonies *in vitro*.

Inhibitors of Notch signaling (such as antibodies that block Notch activation) can be used to inhibit tumorigenic cells. In this manner, the Notch pathway is modified to kill or inhibit the proliferation of tumorigenic cells. For example, an antibody that recognizes Notch 4 blocks the growth of breast cancer tumor cells in vitro and in vivo (see e.g., U.S. Pat. Publ. No. 20020119565 ) and finds use, for example, in preventing tumor metastasis (e.g., after solid tumor removal). Additionally, as shown in Figure 6 and the Examples section below, administration of a y-secretase inhibitors finds use in treating tumorigenic cells, demonstrating that inhibition of the Notch pathway in a manner that does not directly target Notch 4 likewise finds use in the methods disclosed herein. Likewise, as shown in Figure 5, administration of a dominant-negative Mastermindlike-1 adenovirus (dnMAML1Ad), an inhibitor of Notch transcriptional activation, finds use in treating tumorigenic cells.

By contrast, it had previously been found that stimulation of Notch using soluble Delta (Han et al., Blood 95(5): 161625 (2000)), a Notch ligand, promoted growth and survival of tumor cells in vitro. Thus, it had previously been found that stimulation of the Notch pathway promotes growth and survival of the cancer cells.

Experiments conducted during the development of the present invention showed that inhibition of Notch signaling by a dominant-negative Notch inhibitor or a blocking antibody against Notch 4 induced apoptosis via the mitochondrial death pathway in cancer cells from 5 of 5 or from 2 of 3 tumors tested, respectively. In some cases, tumorigenic and non-tumorigenic cancer cells differed in their expression of Notches, Notch ligands, and members of the Fringe family of Notch modifiers. In the tumor where viability of the tumorigenic cancer cells was not affected by the anti-Notch4 antibody, Notch4 expression was detected in the non-tumorigenic but not the tumorigenic cancer cells. Thus, the experiments demonstrate that in some cases of breast cancer, Notch signaling can modulate the growth of the tumorigenic subset of cancer cells and that tumorigenic and non-tumorigenic cancer cells differentially express components of this signaling pathway. These results demonstrate that the prospective identification of tumorigenic cancer cells enables the identification of factors that can affect critical functions of these cells. Since this subpopulation drives tumorigenesis, identification of targets in these cells provides more effective cancer therapies.

The observation that tumors contain a small population of tumorigenic cells with a common cell surface phenotype has important implications for understanding solid tumor biology and also for the development of effective cancer therapies³⁶. The inability of current cancer treatments to cure metastatic disease may be due to ineffective killing of tumorigenic cells. If the tumorigenic cells are spared by an agent, then tumors may regress but the remaining tumorigenic cells will drive tumor recurrence. By focusing on the tumorigenic population, one can identify critical proteins involved in essential biological functions in the tumorigenic population of cancer cells, such as self-renewal and survival. The importance of the prospective identification of the tumorigenic cells for the identification of potential therapeutic targets that might more effectively treat breast cancer is illustrated by T1 and T4, tumor samples obtained from two different subjects. Although both tumors die when Notch signaling is inhibited by the dnMAML1 adenovirus and both tumors express Notch 4, only the T1 clonogenic cells die when exposed to the anti-Notch4 antibody. This can be explained by the observation that the T1 tumorigenic cells expressed Notch4, but expression ofNotch4 could not be detected in the tumorigenic T4 cells, which expressed Notch1 and Notch2. The observation that Notch ligands are expressed at low levels in the tumorigenic cancer cells has implications for tumor biology. It has long been postulated that stromal-cancer cell interactions may play an important role in the growth and metastasis of tumors (reviewed in³⁷). Since Notch activation promotes the growth of clonogenic cells, the expression of Notch ligands by cells in a particular tissue may contribute to the spread of a tumor to that particular site.

Activation of the Notch receptor has previously been implicated in breast cancer and Notch signaling plays a role in transformation of cells transfected with an activated Ras oncogene, but its role in *de novo* human breast cancers is not known¹⁷⁻²⁰. Experiments conducted during the development of the present invention tested the effect of Notch activation in human breast cancer cells isolated from a patient, designated T1 (Tumor 1), by exposing the cells in culture to a soluble form of the Notch ligand Delta, Delta-Fc. Soluble Delta increased five-fold the number of colonies formed by unfractionated T1 cancer cells in culture (Fig. 1a). Next, the effect of inhibition of Notch signaling on the ability of tumorigenic cells isolated from T1 to proliferate *in vitro* was tested by infecting cells with a dominant-negative Mastermindlike-1²¹⁻²⁴ adenovirus (dnMAML1Ad, Fig. 5), an inhibitor of Notch transcriptional activation (Fig.1b). Two days after infection, the dnMAML1Ad, but not a control adenovirus vector, resulted in an 18%-45% decrease in viable T1 tumorigenic cancer cells or clonogenic breast cancer cells from all 4 tumors from other patients (designated T2-T5) that were tested (Fig. 1c). These data show that Notch activation promoted the survival or proliferation of clonogenic cancer cells in all 5 of the de novo tumors that were tested.

The potential role of Notch4 in human breast cancer was examined. Notch4 expression was detected in 4 of the 5 tumors examined, including T1 and T4 (Table 1).

**Table 1. Quantitative RT-PCR gene expression analysis.**

| | T1 | | T4 | | T2 | T3 | T5 |
|---|---|---|---|---|---|---|---|
| | T | NT | T | NT | | | |
| Notch1 | + | +2 | + | + | + | + | + |
| Notch2 | + | + | + | + | - | + | - |
| Notch3 | - | - | - | - | + | - | - |
| Notch4 | + | + | - | + | + | - | + |
| Delta1 | + | +3 | + | +3 | *ND* | *ND* | *ND* |
| Delta3 | - | - | - | - | *ND* | *ND* | *ND* |
| Delta4 | + | +3 | - | - | *ND* | *ND* | *ND* |
| Jagged1 | + | + | + | +3 | *ND* | *ND* | *ND* |
| Lunatic | - | + | - | + | *ND* | *ND* | *ND* |
| Manic | + | - | + | - | *ND* | *ND* | *ND* |
| Radical | - | + | - | + | *ND* | *ND* | *ND* |

T1 cancer cells were cultured in the presence of a polyclonal antibody against Notch4 that inhibited Notch signaling (Fig. 2a). When T1 cancer cells, which expressed Notch4 (Table 1, Fig. 2b), were exposed to this antibody *in vitro,* colony formation was markedly inhibited (Fig. 2c,d). This inhibition was nearly completely eliminated by pre-incubation of the antibody with the Notch4 peptide against which the antibody was generated, confirming the specificity of the anti-Notch4 antibody (Fig. 2c,d). On the other hand, colony formation by the T4 cancer cells was not affected by the anti-Notch 4 antibody (Fig. 2c). Using quantitative RT-PCR, expression of Notch 4 in both T1 and T4 non-tumorigenic cancer cells¹ (Table 1) could be detected. However, when T1 and T4 tumorigenic cells were tested, Notch4 expression was detected in the T1 tumorigenic cancer cell while the T4 tumorigenic cells expressed Notch1 and Notch2, but not Notch4 (Table 1). These data provide an explanation for the observation that clonogenic cells from both tumors die when infected with the dnMAML1 adenovirus (Fig. 1c), but only the T1 clonogenic cells were affected by the anti-Notch4 antibody (Fig. 2c). Further experiments tested whether the anti-Notch4 antibody inhibited tumor formation by tumorigenic T1 cancer cells *in vivo.* As few as 200 T1 tumorigenic cancer cells are able to consistently form tumors in NOD/SCID mice¹. Therefore, 200 T1 tumorigenic cancer cells were incubated with either control buffer or the anti-Notch4 antibody and then injected into mice. 7 of 9 injections of untreated cells and 0 of 9 injections of treated cells formed tumors (Fig. 2e). Tumor formation by larger numbers of antibody-treated cells was delayed relative to control cells.

Further experiments studied the mechanism by which anti-Notch4 antibody inhibited proliferation of the cancer cells in this tumor as well as in tumor cells isolated from additional patients. Notch stimulation has been shown to promote cell proliferation in some circumstances, inhibit proliferation in other circumstances, and to promote cell survival in other cases^{13,25,26}. To distinguish between these possibilities, viability was measured in cells cultured with the anti-Notch4 antibody. After two days of exposure to the antibody, there was a marked increase in cell death of the T1, but not T4, clonogenic cancer cells (Fig. 3a,3b). There were sufficient T1, T4 and T5 tumor cells for further analysis to determine the mechanism by which inhibition of Notch4 signaling induced cell death. Exposure of clonogenic cancer cells isolated from T1 to the anti-Notch4 blocking antibody led to the accumulation of T1 tumorigenic cancer cells with degraded DNA characteristic of apoptosis (Fig. 3a). Compared to control cultures, there was marked increase in the number of cells with activated caspase 3/7 in the T1 and the T5 clonogenic cancer cells, but not the T4 clonogenic cancer cells, 36 hours after antibody exposure (Fig. 3b). These data show that in a subset of *de novo* human breast tumors, Notch pathway activation provides a necessary survival signal to the tumorigenic population of cells.

Programmed cell death can be initiated via either a receptor-mediated (extrinsic) pathway or a mitochondrial (intrinsic) pathway. Unfortunately, it is not currently technically possible to do extensive molecular and biochemical analyses on the rare tumorigenic cancer cells. Therefore, a breast cell lines that depend upon Notch signaling for survival was obtained. MCF-7 cells expressed Notch4 (Fig. 2b), and both the dnMAML1 adenovirus and the anti-Notch4 antibody killed these cells (Fig. 3a, 3c, &3d). As was the case for cells isolated directly from tumors, inhibition of Notch signaling resulted in activation of caspase in the MCF-7 cells (Figure 3b). To determine whether cell death was via the intrinsic or extrinsic cell death pathways, the MCF-7 cells that expressed either a dominant negative (dn) FADD or Bel-x_{L} were used, resulting in inhibition of the intrinsic and extrinsic death pathways respectively (Fig. 3e)^{27,28}. Bel-X_{L}, but not the dnFADD, protected the MCF-7 cells from apoptosis when infected with the dnMAML1 adenovirus (Fig. 3c &3d). These results demonstrate that Notch signaling protects the cancer cells from apoptosis via the mitochondrial death pathway.

Notch signaling is complex. There are multiple Notches and Notch ligands as well as several Fringes that modify these Notches to modulate signaling. Differential expression of various members of the Notch signaling pathway by stem cells and their progenitor cell progeny in normal tissues plays a critical role in stem cell fate decisions^{11,15,29}. Since the differential expression ofNotch4 by T4 tumorigenic and non-tumorigenic cancer cells suggested that a similar situation may also occur in tumors, experiments were conducted to determine whether other Notch pathway genes were differentially expressed in the different populations of cancer cells. To identify the Notch signaling pathway components in breast cancer cells isolated from different patients, quantitative RT-PCR was used to examine the expression of Notches, Notch ligands and Fringes by the cancer cells from each of the 5 tumors. Expression of one or more Notches was detected in all of the tumors (Table 1). In some normal tissues, either stromal cells or differentiated progeny express Notch ligands that provide a paracrine signal that activates Notch in the stem cells^{3,11,30}. Notch signaling is further modulated in normal tissues by expression of the three Fringe proteins, Manic, Lunatic and Radical. Each of the Fringes differentially glycosylates a particular Notch receptor and modulates receptor signaling^{29,31,32}. Experiments were therefore conducted to determine whether tumorigenic and non-tumorigenic cancer cells differed in their expression of Notch ligands and/or Fringe proteins. To accomplish this, quantitative RT-PCR was performed using RNA isolated from 2,000 tumorigenic cancer cells and 2,000 non-tumorigenic cancer cells¹ isolated from T1 and T4 (Fig. 4). Expression of the Notch ligands Delta1 and Delta4 was 3-fold higher in the T1 non-tumorigenic cells as compared to T1 tumorigenic cells while Jagged1 expression was the same in both populations (Table 1). Similarly, the T4 non-tumorigenic cells expressed 3-fold higher levels of the Notch ligands Deltal and Jagged1 than did the tumorigenic cancer cells, while expression of Delta3 and Delta4 was not detected in either population (Table 1).

Since the majority of a tumor consists of non-tumorigenic cancer cells¹ and these cells express higher levels of the Notch ligands than do the tumorigenic cancer cells, these data suggest that the non-tumorigenic cancer cells provide a survival signal to the tumorigenic cancer cells. When examined by quantitative RT-PCR, the expression of Manic Fringe by the tumorigenic cells was detected but not the non-tumorigenic cells in both tumors examined (Table 1). Conversely, expression of Lunatic Fringe and Radical Fringe by the non-tumorigenic cells was detected, but not the tumorigenic cells, in both of the tumors (Table 1). These data are significant since enforced expression of Manic Fringe has been implicated in oncogenic transformation ³³, and in a microarray analysis Manic Fringe expression was highest in normal stem cells³⁴_{.}

The recent observation that the *Bmi*-1 oncogene regulates the self-renewal of botch normal hematopoietic stem cells and leukemia cells firmly links self-renewal pathways to cancer^{8,35}. Since both normal stem cells and a subset of the cancer cells in a tumor share the fundamental ability to self-renew, it is likely that self-renewal pathways are shared by normal stem cells and tumorigenic cancer cells. Therefore, the identification of pathways that regulate self-renewal within the cancer cells is critical to our understanding of these diseases.

The above experiments provide a variety of distinguishing biological markers specific for tumorigenic cells versus non-tumorigenic cells that allow one to characterize the nature of sample, to target via therapeutic intervention, and to assist in selecting and monitoring therapy, drug screening applications, and research applications.

### Therapeutic Agents

A pharmaceutical composition containing a regulator of tumorigenesis according the present invention can be administered by any effective method. The description below relates to an anti-Notch therapeutic agent, which in accordance with the present invention is an anti-Notch antibody. The anti-Notch antibody can be administered by any effective method. For example, a physiologically appropriate solution containing an effective concentration of anti-Notch therapeutic agent can be administered topically, intraocularly, parenterally, orally, intranasally, intravenously, intramuscularly, subcutaneously or by any other effective means. In particular, the anti-Notch therapeutic agent may be directly injected into a target cancer or tumor tissue by a needle in amounts effective to treat the tumor cells of the target tissue. Alternatively, a cancer or tumor present in a body cavity can receive a physiologically appropriate composition (e.g., a solution such as a saline or phosphate buffer, a suspension, or an emulsion, which is sterile) containing an effective concentration of anti-Notch therapeutic agent via direct injection with a needle or via a catheter or other delivery tube placed into the cancer or tumor afflicted hollow organ. Any effective imaging device such as X-ray, sonogram, or fiber-optic visualization system may be used to locate the target tissue and guide the needle or catheter tube. In another alternative, a physiologically appropriate solution containing an effective concentration of anti-Notch therapeutic agent can be administered systemically into the blood circulation to treat a cancer or tumor that cannot be directly reached or anatomically isolated.

All such manipulations have in common the goal of placing the anti-Notch therapeutic agent in sufficient contact with the target tumor to permit the anti-Notch therapeutic agent to contact the tumor cells. In one embodiment, solid tumors present in the epithelial linings of hollow organs may be treated by infusing the vector suspension into a hollow fluid filled organ, or by spraying or misting into a hollow air filled organ. Thus, the tumor cells (such as a solid tumor stem cells) may be present in or among the epithelial tissue in organ tissue accessible to contact with the anti-Notch therapeutic agent. One skilled in the art of oncology can appreciate that the anti-Notch therapeutic agent can be administered to the solid tumor by direct injection into the tumor so that anti-Notch therapeutic agent contacts and affects the tumor cells inside the tumor.

The tumorigenic cells identified by the methods described herein can also be used to raise anti-cancer cell antibodies. The method may involve obtaining an enriched population of tumorigenic cells or isolated tumorigenic cells; treating the population to prevent cell replication (for example, by irradiation); and administering the treated cell to a human or animal subject in an amount effective for inducing an immune response to solid tumor stem cells. For guidance as to an effective dose of cells to be injected or orally administered; see, U.S. Pat. Nos. 6,218,166, 6,207,147, and 6,156,305. Another method involves obtaining an enriched population of solid tumor stem cells or isolated solid tumor stem cells; mixing the tumor stem cells in an in vitro culture with immune effector cells (according to immunological methods known in the art) from a human subject or host animal in which the antibody is to be raised; removing the immune effector cells from the culture; and transplanting the immune effector cells into a host animal in a dose that is effective to stimulate an immune response in the animal.

The therapeutic agent is an antibody (e.g., an anti-Notch4 antibody). Monoclonal antibodies to may be prepared using any technique which provides for the production of antibody molecules by continuous cell lines in culture. These include, but are not limited to, the hybridoma technique, the human B-cell hybridoma technique, and the EBV-hybridoma technique (see, e.g., Kozbor, D. et al., J. Immunol. Methods 81:31-42 (1985); Cote R J et al. Proc. Natl. Acad. Sci. 80:2026-2030 (1983); and Cole S P et al. Mol. Cell Biol. 62:109-120 (1984)).

In addition, techniques developed for the production of "chimeric antibodies," such as the splicing of mouse antibody genes to human antibody genes to obtain a molecule with appropriate antigen specificity and biological activity, can be used (see, e.g., Morrison S L et al. Proc. Natl. Acad. Sci. 81:6851-6855 (1984); Neuberger M S et al. Nature 312:604-608 (1984); and Takeda S et al. Nature 314:452-454 (1985)).

Various immunoassays may be used for screening to identify antibodies having the desired specificity. Numerous protocols for competitive binding or immunoradiometric assays using either polyclonal or monoclonal antibodies with established specificities are well known in the art.

The antibody can also be a humanized antibody. The term "humanized antibody," as used herein, refers to antibody molecules in which the amino acid sequence in the non-antigen binding regions has been altered so that the antibody more closely resembles a human antibody, and still retains its original binding ability. Antibodies are humanized so that they are less immunogenic and therefore persist longer when administered therapeutically to a patient.

Human antibodies can be generated using the XENOMOUSE technology from Abgenix (Fremont, Calif, USA), which enables the generation and selection of high affinity, fully human antibody product candidates to essentially any disease target appropriate for antibody therapy. See, U.S. Pat. Nos. 6,235,883, 6,207,418,6,162,963, 6,150,584, 6,130,364,6,114,598, 6,091,001, 6,075,181, 5,998,209, 5,985,615, 5,939,598, and 5,916,771; Yang X et al., Crit Rev Oncol Hemato 38(1): 17-23 (2001); Chadd H E & Chamow S M. Curr Opin Biotechnol 12(2):188-94 (2001); Green L L, Journal of Immunological Methods 231 11-23 (1999); Yang X-D et al., Cancer Research 59(6): 1236-1243 (1999); and Jakobovits A, Advanced Drug Delivery Reviews 31: 33-42 (1998). Antibodies with fully human protein sequences are generated using genetically engineered strains of mice in which mouse antibody gene expression is suppressed and functionally replaced with human antibody gene expression, while leaving intact the rest of the mouse immune system. Moreover, the generation of antibodies directed against markers present in or on the tumorigenic cells can be used as a method of identifying targets for drug development.

In the present invention, the anti-tumorigenic therapeutic agents of the present invention are co-adminstered with other anti-neoplastic therapies, as is defined by the claims. Chemo-therapeutic agents find use with the present invention. Any chemo-therapeutic agent that can be co-administered with the agents of the present invention, or associated with the agents of the present invention is suitable for use in the methods of the present invention.

Accordingly, an anti-Notch antibody of the present invention is provided for administering with at least one chemotherapeutic antineoplastic and optionally a therapeutic technique (*e.g*., surgical intervention, radiotherapies).

In the present invention, the anti-Notch antibody is for use in combination with an anticancer agent which is a chemotherapeutic agent as defined by the claims. These and other anticancer agents are described below. Anticancer agents include, but are not limited to, agents that induce apoptosis, agents that inhibit adenosine deaminase function, inhibit pyrimidine biosynthesis, inhibit purine ring biosynthesis, inhibit nucleotide interconversions, inhibit ribonucleotide reductase, inhibit thymidine monophosphate (TMP) synthesis, inhibit dihydrofolate reduction, inhibit DNA synthesis, form adducts with DNA, damage DNA, inhibit DNA repair, intercalate with DNA, deaminate asparagines, inhibit RNA synthesis, inhibit protein synthesis or stability, inhibit microtubule synthesis or function, and the like.

Exemplary anticancer agents include, but are not limited to: 1) alkaloids, including microtubule inhibitors (*e.g*., vincristine, vinblastine, and vindesine, *etc*.), microtubule stabilizers (*e.g*., paclitaxel (TAXOL), and docetaxel, *etc*.), and chromatin function inhibitors, including topoisomerase inhibitors, such as epipodophyllotoxins (*eg*., etoposide (VP-16), and teniposide (VM-26), *etc*.), and agents that target topoisomerase I (*e.g*., camptothecin and isirinotecan (CPT-11), *etc*.); 2) covalent DNA-binding agents (alkylating agents), including nitrogen mustards (*e.g*., mechlorethamine, chlorambucil, cyclophosphamide, ifosphamide, and busulfan (MYLERAN), *etc*.), nitrosoureas (*e.g*., Carmustine, lomustine, and semustine, *etc*.), and other alkylating agents (*e.g*., dacarbazine, hydroxymethylmelamine, thiotepa, and mitomycin, *etc*.); 3) noncovalent DNA-binding agents (antitumor antibiotics), including nucleic acid inhibitors (*e.g*., dactinomycin (actinomycin D), *etc*.), anthracyclines (*e.g*., daunorubicin (daunomycin, and cerubidine), doxorubicin (adriamycin), and idarubicin (idamycin), *etc*.), anthracenediones (*e.g*., anthracycline analogues, such as mitoxantrone, *etc*.), bleomycins (BLENOXANE), *etc*., and plicamycin (mithramycin), *etc*.; *4*) antimetabolites, including antifolates (*e.g*., methotrexate, FOLEX, and MEXATE, *etc*.), purine antimetabolites (*e.g*., 6-mercaptopurine (6-MP, PURINETHOL), 6-thioguanine (6-TG), azathioprine, acyclovir, ganciclovir, chlorodeoxyadenosine, 2-chlorodeoxyadenosine (CdA), and 2'-deoxycoformycin (pentostatin), *etc*.), pyrimidine antagonists (*e.g*., fluoropyrimidines (*e.g*., 5-fluorouracil (ADRUCIL), 5-fluorodeoxyuridine (FdUrd) (floxuridine)) *etc*.), and cytosine arabinosides (*e.g*., CYTOSAR (ara-C) and fludarabine, *etc*.); 5) enzymes, including L-asparaginase, and hydroxyurea, *etc*.; 6) hormones, including glucocorticoids, antiestrogens (*e.g*., tamoxifen, *etc*.), nonsteroidal antiandrogens (*e.g*., flutamide, *etc*.), and aromatase inhibitors (*e.g*., anastrozole (ARIMIDEX), *etc.*); 7) platinum compounds (*e.g*., cisplatin and carboplatin, *etc*.); 8) monoclonal antibodies conjugated with anticancer drugs, toxins, and/or radionuclides, *etc*.; 9) biological response modifiers (*e.g*., interferons (*e.g*., IFN-α, *etc*.) and interleukins (*e.g*., IL-2, *etc*.), *etc*.); 10) adoptive immunotherapy; 11) hematopoietic growth factors; 12) agents that induce tumor cell differentiation (*e.g*., all-trans-retinoic acid, *etc*.); 13) gene therapy techniques; 14) antisense therapy techniques; 15) tumor vaccines; 16) therapies directed against tumor metastases (*e.g*., batimastat, *etc*.); 17) angiogenesis inhibitors; 18) proteosome inhibitors (*e.g*., VELCADE); 19) inhibitors of acetylation and/or methylation (*e.g*., HDAC inhibitors); 20) modulators of NF kappa B; 21) inhibitors of cell cycle regulation (*e.g*., CDK inhibitors); 22) modulators of p53 protein function; and 23) radiation.

Any oncolytic agent that is routinely used in a cancer therapy context finds use in the compositions and methods of the present invention. For example, the U.S. Food and Drug Administration maintains a formulary of oncolytic agents approved for use in the United States. International counterpart agencies to the U.S.F.D.A. maintain similar formularies. Table X provides a list of exemplary antineoplastic agents approved for use in the U.S. Those skilled in the art will appreciate that the "product labels" required on all U.S. approved chemotherapeutics describe approved indications, dosing information, toxicity data, and the like, for the exemplary agents.

| | | |
|---|---|---|
| Aldesleukin (des-alanyl-1, serine-125 human interleukin-2) | Proleukin | Chiron Corp., Emeryville, CA |
| Alemtuzumab (IgG1κ anti CD52 antibody) | Campath | Millennium and ILEX Partners, LP, Cambridge, MA |
| Alitretinoin (9-cis-retinoic acid) | Panretin | Ligand Pharmaceutical, Inc., San Diego CA |
| Allopurinol (1,5-dihydro-4 H pyrazolo[3,4-d]pyrimidin-4-one monosodium salt) | Zyloprim | GlaxoSmithKline, Research Triangle Park, NC |
| Altretamine (N,N,N',N',N",N",- hexamethyl-1,3,5-triazine-2, 4, 6-triamine) | Hexalen | US Bioscience, West Conshohocken, PA |
| Amifostine (ethanethiol, 2-[(3-aminopropyl)amino]-, dihydrogen phosphate (ester)) | Ethyol | US Bioscience |
| Anastrozole (1,3-Benzenediacetonitrile, a, a, a', a'-tetramethyl-5-(1H-1,2,4-triazol-1-ylmethyl)) | Arimidex | AstraZeneca Pharmaceuticals, LP, Wilmington, DE |
| Arsenic trioxide | Trisenox | Cell Therapeutic, Inc., Seattle, WA |
| Asparaginase (L-asparagine amidohydrolase, type EC-2) | Elspar | Merck & Co., Inc., Whitehouse Station, NJ |
| BCG Live (lyophilized preparation of an attenuated strain of *Mycobacterium bovis (Bacillus Calmette-Gukin* [BCG], substrain Montreal) | TICE BCG | Organon Teknika, Corp., Durham, NC |
| bexarotene capsules (4-[1-(5,6,7,8-tetrahydro-3,5,5,8,8-pentamethyl-2-napthalenyl) ethenyl] benzoic acid) | Targretin | Ligand Pharmaceuticals |
| bexarotene gel | Targretin | Ligand Pharmaceuticals |
| Bleomycin (cytotoxic glycopeptide antibiotics produced by *Streptomyces verticillus;* bleomycin A₂ and bleomycin B₂) | Blenoxane | Bristol-Myers Squibb Co., NY, NY |
| Capecitabine (5'-deoxy-5-fluoro-N-[(pentyloxy)carbonyl]-cytidine) | Xeloda | Roche |
| Carboplatin (platinum, diammine [1,1-cyclobutanedicarboxylato(2-)-0, 0']-,(SP-4-2)) | Paraplatin | Bristol-Myers Squibb |
| Carmustine (1,3-bis(2-chloroethyl)-1-nitrosourea) | BCNU, BiCNU | Bristol-Myers Squibb |
| Carmustine with Polifeprosan 20 Implant | Gliadel Wafer | Guilford Pharmaceuticals, Inc., Baltimore, MD |
| Celecoxib (as 4-[5-(4-methylphenyl)-3- (trifluoromethyl)-1H-pyrazol-1-yl] benzenesulfonamide) | Celebrex | Searle Pharmaceuticals, England |
| Chlorambucil (4-[bis(2chlorethyl)amino]benzenebutanoic acid) | Leukeran | GlaxoSmithKline |
| Cisplatin (PLCl₂H₆N₂) | Platinol | Bristol-Myers Squibb |
| Cladribine (2-chloro-2'-deoxy-b-D-adenosine) | Leustatin, 2-CdA | R.W. Johnson Pharmaceutical Research Institute, Raritan, NJ |
| Cyclophosphamide (2-[bis(2-chloroethyl)amino] tetrahydro-2H-13,2-oxazaphosphorine 2-oxide monohydrate) | Cytoxan, Neosar | Bristol-Myers Squibb |
| Cytarabine (1-b-D-Arabinofuranosylcytosine, C₉H₁₃N₃O₅) | Cytosar-U | Pharmacia & Upjohn Company |
| Cytarabine liposomal | DepoCyt | Skye Pharmaceuticals, Inc., San Diego, CA |
| Dacarbazine (5-(3,3-dimethyl-1-triazeno)-imidazole-4-carboxamide (DTIC)) | DTIC-Dome | Bayer AG, Leverkusen, Germany |
| Dactinomycin, actinomycin D (actinomycin produced by *Streptomyces parvullus,* C₆₂H₈₆N₁₂O₁₆) | Cosmegen | Merck |
| Darbepoetin alfa (recombinant peptide) | Aranesp | Amgen, Inc., Thousand Oaks, CA |
| daunorubicin liposomal ((8S-cis)-8-acetyl-10-[(3-amino-2,3,6-trideoxy-á-L-lyxo-hexopyranosyl)oxy]-7,8,9,10-tetrahydro-6,5,11-trihydroxy-1-methoxy-5,12-naphthacenedione hydrochloride) | DanuoXome | Nexstar Pharmaceuticals, Inc., Boulder, CO |
| Daunorubicin HCl, daunomycin ((1*S* ,3*S*)-3-Acetyl-1,2,3,4,6,11-hexahydro-3,5,12-trihydroxy-10-methoxy-6,11-dioxo-1-naphthacenyl 3-amino-2,3,6-trideoxy-(alpha)-L-*lyxo* -hexopyranoside hydrochloride) | Cerubidine | Wyeth Ayerst, Madison, NJ |
| Denileukin diftitox (recombinant peptide) | Ontak | Seragen, Inc., Hopkinton, MA |
| Dexrazoxane ((*S*)-4,4'-(1-methyl-1,2-ethanediyl)bis-2,6-piperazinedione) | Zinecard | Pharmacia & Upjohn Company |
| Docetaxel ((2R,3S)-N-carboxy-3-phenylisoserine, N-tert-butyl ester, 13-ester with 5b-20-epoxy-12a,4,7b,10b,13a-hexahydroxytax- 11-en-9-one 4-acetate 2-benzoate, trihydrate) | Taxotere | Aventis Pharmaceuticals, Inc., Bridgewater, NJ |
| Doxorubicin HCl (8S,10S)-10-[(3-amino-2,3,6-trideoxy-a-L-lyxo-hexopyranosyl)oxy] -8-glycolyl-7,8,9,10-tetrahydro-6,8,11- trihydroxy-1-methoxy-5,12-naphthacenedione hydrochloride) | Adriamycin, Rubex | Pharmacia & Upjohn Company |
| doxorubicin | Adriamycin PFS Intravenous injection | Pharmacia & Upjohn Company |
| doxorubicin liposomal | Doxil | Sequus Pharmaceuticals, Inc., Menlo park, CA |
| dromostanolone propionate (17b-Hydroxy-2a-methyl-5a-androstan-3-one propionate) | Dromostanolone | Eli Lilly & Company, Indianapolis, IN |
| dromostanolone propionate | Masterone injection | Syntex, Corp., Palo Alto, CA |
| Elliott's B Solution | Elliott's B Solution | Orphan Medical, Inc |
| Epirubicin ((8S-cis)-10-[(3-amino-2,3,6-trideoxy-a-L-arabino- hexopyranosyl)oxy]-7,8,9,10-tetrahydro-6,8,11-trihydroxy-8- (hydroxyacetyl)-1-methoxy-5,12-naphthacenedione hydrochloride) | Ellence | Pharmacia & Upjohn Company |
| Epoetin alfa (recombinant peptide) | Epogen | Amgen, Inc |
| Estramustine (estra-1,3,5(10)-triene-3,17-diol(17(beta))-, 3-[bis(2-chloroethyl)carbamate] 17-(dihydrogen phosphate), disodium salt, monohydrate, or estradiol 3-[bis(2-cbloroethyl)carbamate] 17-(dihydrogen phosphate), disodium salt, monohydrate) | Emcyt | Pharmacia & Upjohn Company |
| Etoposide phosphate (4'-Demethylepipodophyllotoxin 9-[4,6-O-(R)-ethylidene-(beta)-D-glucopyranoside], 4'-(dihydrogen phosphate)) | Etopophos | Bristol-Myers Squibb |
| etoposide, VP-16 (4'-demethylepipodophyllotoxin 9-[4,6-0-(R)-ethylidene-(beta)-D-glucopyranoside]) | Vepesid | Bristol-Myers Squibb |
| Exemestane (6-methylenandrosta-1,4-diene-3, 17-dione) | Aromasin | Pharmacia & Upjohn Company |
| Filgrastim (r-metHuG-CSF) | Neupogen | Amgen, Inc |
| floxuridine (intraarterial) (2'-deoxy-5-fluorouridine) | FUDR | Roche |
| Fludarabine (fluorinated nucleotide analog of the antiviral agent vidarabine, 9-b -D-arabinofuranosyladenine (ara-A)) | Fludara | Berlex Laboratories, Inc., Cedar Knolls, NJ |
| Fluorouracil, 5-FU (5-fluoro-2,4(1H,3H)-pyrimidinedione) | Adrucil | ICN Pharmaceuticals, Inc., Humacao, Puerto Rico |
| Fulvestrant (7-alpha-[9-(4,4,5,5,5-penta fluoropentylsulphinyl) nonyl]estra-1,3,5-(10)- triene-3,17-beta-diol) | Faslodex | IPR Pharmaceuticals, Guayama, Puerto Rico |
| Gemcitabine (2'-deoxy-2', 2'-difluorocytidine monohydrochloride (b-isomer)) | Gemzar | Eli Lilly |
| Gemtuzumab Ozogamicin (anti-CD33 hP67.6) | Mylotarg | Wyeth Ayerst |
| Goserelin acetate (acetate salt of [D-Ser(But)⁶,Azgly¹⁰]LHRH; pyro-Glu-His-Trp-Ser-Tyr-D-Ser(But)-Leu-Arg-Pro-Azgly-NH2 acetate [C₅₉H₈₄N₁₈O₁₄ •(C₂H₄O₂)_{X} | Zoladex Implant | AstraZeneca Pharmaceuticals |
| Hydroxyurea | Hydrea | Bristol-Myers Squibb |
| Ibritumomab Tiuxetan (immunoconjugate resulting from a thiourea covalent bond between the monoclonal antibody Ibritumomab and the linker-chelator tiuxetan [N-[2-bis(carboxymethyl)amino]-3-(p-isothiocyanatophenyl)- propyl]-[N-[2-bis(carboxymethyl)amino]-2-(methyl) -ethyl]glycine) | Zevalin | Biogen IDEC, Inc., Cambridge MA |
| Idarubicin (5, 12-Naphthacenedione, 9-acetyl-7-[(3-amino-2,3,6-trideoxy-(alpha)-L-lyxo -hexopyranosyl)oxy]-7,8,9,10-tetrahydro-6,9,11-trihydroxyhydrochloride, (7S- cis)) | Idamycin | Pharmacia & Upjohn Company |
| Ifosfamide (3-(2-chloroethyl)-2-[(2-chloroethyl)amino]tetrahydro-2H-1,3,2-oxazaphosphorine 2-oxide) | IFEX | Bristol-Myers Squibb |
| Imatinib Mesilate (4-[(4-Methyl-1-piperazinyl)methyl]-N-[4-methyl-3-[[4-(3-pyridinyl)-2-pyrimidinyl]amino]-phenyl]benzamide methanesulfonate) | Gleevec | Novartis AG, Basel, Switzerland |
| Interferon alfa-2a (recombinant peptide) | Roferon-A | Hoffmann-La Roche, Inc., Nutley, NJ |
| Interferon alfa-2b (recombinant peptide) | Intron A (Lyophilized Betaseron) | Schering AG, Berlin, Germany |
| Irinotecan HCl ((4S)-4,11-diethyl-4-hydroxy-9-[(4- piperi-dinopiperidino)carbonyloxy]-1H-pyrano[3', 4': 6,7] indolizino[1,2-b] quinoline-3,14(4H, 12H) dione hydrochloride trihydrate) | Camptosar | Pharmacia & Upjohn Company |
| Letrozote (4,4'-(1H-1,2,4 -Triazol-1-ylmethylene) dibenzonitrile) | Femara | Novartis |
| Leucovorin (L-Glutamic acid, N[4[[(2amino-5-formyl-1,4,5,6,7,8 hexahydro4oxo6-pteridinyl)methyl]amino]benzoyl], calcium salt (1:1)) | Wellcovorin, Leucovorin | Immunex, Corp., Seattle, WA |
| Levamisole HCl ((-)-(S)-2,3,5, 6-tetrahydro-6-phenylimidazo [2,1-b] thiazole monohydrochloride C₁₁R₁₂N₂S· HCl) | Ergamisol | Janssen Research Foundation, Titusville, NJ |
| Lomustine (1-(2-chloro-ethyl)-3-cyclohexyl-1-nitrosourea) | CeeNU | Bristol-Myers Squibb |
| Meclorethamine, nitrogen mustard (2-chloro-N-(2-chloroethyl)-N-methylethanamine hydrochloride) | Mustargen | Merck |
| Megestrol acetate 17α(acetyloxy)- 6- methylpregna- 4,6- diene-3,20- dione | Megace | Bristol-Myers Squibb |
| Melphalan, L-PAM (4-[bis(2-chloroethyl) amino]-L-phenylalanine) | Alkeran | GlaxoSmithKline |
| Mercaptopurine, 6-MP (1,7-dihydro-6 H purine-6-thione monohydrate) | Purinethol | GlaxoSmithKline |
| Mesna (sodium 2-mercaptoethane sulfonate) | Mesnex | Asta Medica |
| Methotrexate (N-[4-[[(2,4-diamino-6-pteridinyl)methyl]methylamino]benzoyl]-L-glutamic acid) | Methotrexate | Lederle Laboratories |
| Methoxsalen (9-methoxy-7H-furo[3,2-g][1]-benzopyran-7-one) | Uvadex | Therakos, Inc., Way Exton, Pa |
| Mitomycin C | Mutamycin | Bristol-Myers Squibb |
| mitomycin C | Mitozytrex | SuperGen, Inc., Dublin, CA |
| Mitotane (1,1-dichloro-2-(o-chlorophenyl)-2-(p-chlorophenyl) ethane) | Lysodren | Bristol-Myers Squibb |
| Mitoxantrone (1,4-dihydroxy-5,8-bis[[2- [(2-hydroxyethyl)amino]ethyl]amino]-9,10-anthracenedione dihydrochloride) | Novantrone | Immunex Corporation |
| Nandrolone phenpropionate | Durabolin-50 | Organon, Inc., West Orange, NJ |
| Nofetumomab | Verluma | Boehringer Ingelheim Pharma KG, Germany |
| Oprelvekin (IL-11) | Neumega | Genetics Institute, Inc., Alexandria, VA |
| Oxaliplatin (cis-[(1R,2R)-1,2-cyclohexanediamine-N,N'] [oxalato(2-)-O,O'] platinum) | Eloxatin | Sanofi Synthelabo, Inc., NY, NY |
| Paclitaxel (5B, 20-Epoxy-1,2a, 4,7B, 10B, 13a-hexahydroxytax-11-en-9-one 4,10-diacetate 2-benzoate 13-ester with (2R, 3 S)- N-benzoyl-3-phenylisoserine) | TAXOL | Bristol-Myers Squibb |
| Pamidronate (phosphonic acid (3-amino-1-hydroxypropylidene) bis-, disodium salt, pentahydrate, (APD)) | Aredia | Novartis |
| Pegademase ((monomethoxypolyethylene glycol succinimidyl) 11 - 17 -adenosine deaminase) | Adagen (Pegademase Bovine) | Enzon Pharmaceuticals, Inc., Bridgewater, NJ |
| Pegaspargase (monomethoxypolyethylene glycol succinimidyl L-asparaginase) | Oncaspar | Enzon |
| Pegfilgrastim (covalent conjugate of recombinant methionyl human G-CSF (Filgrastim) and monomethoxypolyethylene glycol) | Neulasta | Amgen, Inc |
| Pentostatin | Nipent | Parke-Davis Pharmaceutical Co., Rockville, MD |
| Pipobroman | Vercyte | Abbott Laboratories, Abbott Park, IL |
| Plicamycin, Mithramycin (antibiotic produced by *Streptomyces plicatus*) | Mithracin | Pfizer, Inc., NY, NY |
| Porfimer sodium | Photofrin | QLT Phototherapeutics, Inc., Vancouver, Canada |
| Procarbazine (N-isopropyl-µ-(2-methylhydrazino)-p-toluamide monohydrochloride) | Matulane | Sigma Tau Pharmaceuticals, Inc., Gaithersburg, MD |
| Quinacrine (6-chloro-9-(1-methyt-4-diethyl-amine) butylamino-2-methoxyacridine) | Atabrine | Abbott Labs |
| Rasburicase (recombinant peptide) | Elitek | Sanofi-Synthelabo, Inc., |
| Rituximab (recombinant anti-CD20 antibody) | Rituxan | Genentech, Inc., South San Francisco, CA |
| Sargramostim (recombinant peptide) | Prokine | Immunex Corp |
| Streptozocin (streptozocin 2 -deoxy - 2 -[[(methylnitrosoamino)carbonyl]amino] - a(and b) - D - glucopyranose and 220 mg citric acid anhydrous) | Zanosar | Pharmacia & Upjohn Company |
| Talc (Mg₃Si₄O₁₀ (OH)₂) | Sclerosol | Bryan, Corp., Woburn, MA |
| Tamoxifen ((Z)2-[4-(1,2-diphenyl-1-butenyl) phenoxy]-N, N-dimethylethanamine 2-hydroxy-1,2,3-propanetricarboxylate (1:1)) | Nolvadex | AstraZeneca Pharmaceuticals |
| Temozolomide (3,4-dihydro-3-methyl-4-oxoimidazo[5,1-d]-as-tetrazine-8-carboxamide) | Temodar | Schering |
| teniposide, VM-26 (4'-demethylepipodophyllotoxin 9-[4,6-0-(R)-2-thenylidene-(beta)-D-glucopyranoside]) | Vumon | Bristol-Myers Squibb |
| Testolactone (13-hydroxy-3-oxo-13,17-secoandrosta-1,4-dien-17-oic acid [dgr]-lactone) | Teslac | Bristol-Myers Squibb |
| Thioguanine, 6-TG (2-amino-1,7-dihydro-6H - purine-6-thione) | Thioguanine | GlaxoSmithKline |
| Thiotepa (Aziridine, 1,1',1"-phosphinothioylidynetris-, or Tris (1-aziridinyl) phosphine sulfide) | Thioplex | Immunex Corporation |
| Topotecan HCl ((S)-10-[(dimethylamino) methyl]-4-ethyl-4,9-dihydroxy-1H-pyrano[3', 4': 6,7] indolizino [1,2-b] quinoline-3,14-(4H,12H)-dione monohydrochloride) | Hycamtin | GlaxoSmithKline |
| Toremifene (2-(p-[(Z)-4-chloro-1,2-diphenyl-1-butenyl]-phenoxy)-N,N-dimethylethylamine citrate (1:1)) | Fareston | Roberts Pharmaceutical Corp., Eatontown, NJ |
| Tositumomab, I 131 Tositumomab (recombinant murine immunotherapeutic monoclonal IgG₂ₐ lambda anti-CD20 antibody (I 131 is a radioimmunotherapeutic antibody)) | Bexxar | Corixa Corp., Seattle, WA |
| Trastuzumab (recombinant monocional IgG₁ kappa anti-HER2 antibody) | Herceptin | Genentech, Inc |
| Tretinoin, ATRA (all-trans retinoic acid) | Vesanoid | Roche |
| Uracil Mustard | Uracil Mustard Capsules | Roberts Labs |
| Valrubicin, N-trifluoroacetyladriamycin-14-valerate ((25-cis)-2- [1,2,3,4,6,11-hexahydro-2,5,12-trihydroxy-7 methoxy-6,11-dioxo-[[4 2,3,6-trideoxy-3- [(trifluoroacetyl)-amino-α-L-*lyxo*-hexopyranosyl]oxyl]-2-naphthacenyl]-2-oxoethyl pentanoate) | Valstar | Anthra --> Medeva |
| Vinblastine, Leuroctistine (C₄₆H₅₆N₄O₁₀·H₂SO₄) | Velban | Eli Lilly |
| Vincristine (C₄₆H₅₆N₄O₁₀·H₂SO₄) | Oncovin | Eli Lilly |
| Vinorelbine (3' ,4'-didehydro-4'-deoxy-C'-norvincaleukoblastine [R-(R*,R*)-2,3-dihydroxybutanedioate (1:2)(salt)]) | Navelbine | GlaxoSmithKline |
| Zoledronate, Zoledronic acid ((1-Hydroxy-2-imidazol-1-yl-phosphonoethyl) phosphonic acid monohydrate) | Zometa | Novartis |

Antimicrobial therapeutic agents may also be used as therapeutic agents in the present invention. Any agent that can kill, inhibit, or otherwise attenuate the function of microbial organisms may be used, as well as any agent contemplated to have such activities. Antimicrobial agents include, but are not limited to, natural and synthetic antibiotics, antibodies, inhibitory proteins (*e.g*., defensins), antisense nucleic acids, membrane disruptive agents and the like, used alone or in combination. Indeed, any type of antibiotic may be used including, but not limited to, antibacterial agents, antiviral agents, antifungal agents, and the like.

Disclosed herein are therapeutic compounds associated with targeting agents that are able to specifically target particular cell types (*e.g*., tumor cells). Generally, the therapeutic compound that is associated with a targeting agent, targets neoplastic cells through interaction of the targeting agent with a cell surface moiety that is taken into the cell through receptor mediated endocytosis.

Any moiety known to be located on the surface of target cells (*e.g*., tumor cells) finds use. For example, an antibody directed against such a moiety targets the compositions to cell surfaces containing the moiety. Alternatively, the targeting moiety may be a ligand directed to a receptor present on the cell surface or vice versa. Similarly, vitamins also may be used to target the therapeutics to a particular cell.

As used herein, the term "targeting molecules" refers to chemical moieties, and portions thereof useful for targeting therapeutic compounds to cells, tissues, and organs of interest. Various types of targeting molecules are contemplated including, but not limited to, signal peptides, antibodies, nucleic acids, toxins and the like. Targeting moieties may additionally promote the binding of the associated chemical compounds (*e.g*., small molecules) or the entry of the compounds into the targeted cells, tissues, and organs. Preferably, targeting moieties are selected according to their specificity, affinity, and efficacy in selectively delivering attached compounds to targeted sites within a subject, tissue, or a cell, including specific subcellular locations and organelles.

Various efficiency issues affect the administration of all drugs--and of highly cytotoxic drugs (*e.g*., anticancer drugs) in particular. One issue of particular importance is ensuring that the administered agents affect only targeted cells (*e.g*., cancer cells), tissues, or organs. The nonspecific or unintended delivery of highly cytotoxic agents to nontargeted cells can cause serious toxicity issues.

Numerous attempts have been made to devise drug-targeting schemes to address the problems associated with nonspecific drug delivery. (*See e.g.,* K.N. Syrigos and A.A. Epenetos Anticancer Res., 19:606-614 (1999); Y.J. Park et al., J. Controlled Release, 78:67-79 (2002); R.V.J. Chari, Adv. Drug Deliv. Rev., 31:89-104 (1998); and D. Putnam and J. Kopecek, Adv. Polymer Sci., 122:55-123 (1995)). Conjugating targeting moieties such as antibodies and ligand peptides (*e.g*., RDG for endothelium cells) to drug molecules has been used to alleviate some collateral toxicity issues associated with particular drugs. However, conjugating drugs to targeting moieties alone does not completely negate potential side effects to nontargeted cells, since the drugs are usually bioactive on their way to target cells. Advances in targeting moiety-prodrug conjugates, which are inactive while traveling to specific targeted tissues, have diminished some of these concerns. A biotransformation, such as enzymatic cleavage, typically converts the prodrug into a biologically active molecule at the target site.

Accordingly, disclosed herein are prodrug conjugates that are inactive until they reach their target site, where they are subsequently converted into an active therapeutic drug molecule. ADEPT and ATTEMPTS are two exemplary prodrug delivery systems . (See K.N. Syrigos and A.A. Epenetos, Anticancer Res., 19:606-614 (1999); K.D. Bagshawe, Brit. J. Cancer, 56:531-532 (1987); Y.J. Park et al., J. Controlled Release, 72:145-156 (2001); and Y.J. Park et al., J. Controlled Release, 78:67-79 (2002)).

The rapid clearance of some types of therapeutic agents, especially water-soluble low-molecular weight agents, from the subject's bloodstream provides yet another obstacle to effective small molecule administration. Still other obstacles come from the rapid clearance (*e.g*., proteolytic degradation) or potential immunogenicity of the administered agents.

In natural systems, clearance and other pharmacokinetic behaviors of small molecules (*e.g*., drugs) in a subject are regulated by a series of transport proteins. (*See e.g.,* H.T. Nguyen, Clin. Chem. Lab. Anim., (2nd Ed.) pp. 309-335 (1999); and G.J. Russell-Jones and D.H. Alpers, Pharm. Biotechnol., 12:493-520 (1999)). Thus, , the pharmacokinetics of agents are considered when testing and developing potential therapeutics.

The rate of agent clearance in a subject is typically manageable. For instance, attaching (*e.g*., binding) the agent to a macromolecular carrier normally prolongs circulation and retention times. Accordingly, small molecules may be conjugated to polyethylene glycol (PEG), or similar biopolymers, to decrease (prevent) the molecules' degradation and to improve its retention in the subject's bloodstream. *(See e.g.,* R.B. Greenwald et al., Critical Rev. Therapeutic Drug Carrier Syst., 17:101-161 (2000)). The ability of PEG to discourage protein-protein interactions can reduce the immunogenicity of many drugs.

Another issue affecting the administration of some therapeutic agents, and especially hydrophilic and macromolecular drugs such as peptides and nucleic acids, is that these agents have difficulty crossing into targeted cellular membranes. Small (typically less than 1,000 Daltons) hydrophobic molecules are less susceptible to having difficulties entering target cell membranes. Moreover, low molecular weight cytotoxic drugs often localize more efficiently in normal tissues rather than in target tissues such as tumors (K. Bosslet et al., Cancer Res., 58:1195-1201 (1998)) due to the high interstitial pressure and unfavorable blood flow properties within rapidly growing tumors (R.K. Jain, Int. J. Radiat. Biol., 60:85-100 (1991); and R.K. Jain and L.T. Baxter, Cancer Res., 48:7022-7032 (1998)).

The following methods or compositions may be used to aid delivery of the therapeutic compositions, especially of cytotoxic agents : microinjection (*See e.g*., M. Foldvari and M. Mezei, J. Pharm. Sci., 80:1020-1028, (1991)); scrape loading (*See e.g.,* P.L. McNeil et al., J. Cell Biol., 98:1556-1564 (1984)); electroporation (*See e.g.,* R. Chakrabarti et al., J. Biol. Chem., 26:15494-15500 (1989)); liposomes (*See e.g.,* M. Foldvari et al., J. Pharm. Sci., 80:1020-1028 (1991); and J.N. Moreira et al., Biochim Biophys Acta., 515:167-176 (2001)); nanocarriers such as water-soluble polymers (*e.g*., enhanced permeation and retention "EPR", *See e.g.,* H. Maeda et al., J. Controlled Release, 65:271-284 (2000); H. Maeda *et al.,* supra; and L.W. Seymour, Crit. Rev. Therapeu. Drug Carrier Systems, 9:135-187 (1992)); bacterial toxins *(See e.g.,* T.I. Prior et al., Biochemistry, 31:3555-3559 (1992); and H. Stenmark et al., J. Cell Biol., 113:1025-1032 (1991)); receptor-mediated endocytosis and phagocytosis, including the tumor-activated prodrug (TAP) system (*See e.g.,* R.V.J. Chari, Adv. Drug Deliv. Rev., 31:89-104 (1998); I. Mellman, Annu. Rev. Cell Dev. Biol., 12:575-625 (1996); C.P. Leamon and P.S. Low, J. Biol. Chem., 267 (35):24966-24971 (1992); H. Ishihara et al., Pharm. Res., 7:542-546 (1990); S.K. Basu, Biochem. Pharmacol., 40:1941-1946 (1990); and G.Y. Wu and C.H. Wu, Biochemistry, 27:887-892 (1988)); other suitable compositions and methods are known in the art.

The compounds and anticancer agents may be administered in any sterile, biocompatible pharmaceutical carrier, including, but not limited to, saline, buffered saline, dextrose, and water. The pharmaceutical compositions disclosed herein may contain one agent (*e.g*., an antibody). The pharmaceutical compositions may also contain a mixture of at least two agents (*e.g*., an antibody and one or more conventional anticancer agents). The pharmaceutical compositions disclosed may contain at least two agents that are administered to a patient under one or more of the following conditions: at different periodicities, at different durations, at different concentrations, by different administration routes, *etc.* The therapeutic compound may be administered prior to the anticancer agent, *e.g*., 0.5, 1, 2 3, 4, 5, 10, 12, or 18 hours, 1, 2, 3, 4, 5, or 6 days, 1, 2, 3, or 4 weeks prior to the administration of the anticancer agent. The therapeutic compound may be administered after the anticancer agent, *e.g*., 0.5, 1, 2 3, 4, 5, 10, 12, or 18 hours, 1, 2, 3, 4, 5, or 6 days, 1, 2, 3, or 4 weeks after the administration of the anticancer agent. The therapeutic compound and the anticancer agent may be administered concurrently but on different schedules, *e.g*., the therapeutic compound is administered daily while the anticancer agent is administered once a week, once every two weeks, once every three weeks, or once every four weeks. The therapeutic compound may be administered once a week while the anticancer agent is administered daily, once a week, once every two weeks, once every three weeks, or once every four weeks.

Depending on the condition being treated, preferred embodiments of the present pharmaceutical compositions are formulated and administered systemically or locally. Techniques for formulation and administration can be found in the latest edition of "Remington's Pharmaceutical Sciences" (Mack Publishing Co, Easton Pa.). Suitable routes may, for example, include oral or transmucosal administration as well as parenteral delivery (*e.g*., intramuscular, subcutaneous, intramedullary, intrathecal, intraventricular, intravenous, intraperitoneal, or intranasal administration).

Disclosed herein is the administration of therapeutic compounds and one or more conventional anticancer agents, in accordance with acceptable pharmaceutical delivery methods and preparation techniques. For example, therapeutic compounds and suitable anticancer agents can be administered to a subject intravenously in a pharmaceutically acceptable carrier such as physiological saline. Standard methods for intracellular delivery of pharmaceutical agents are contemplated (*e.g*., delivery via liposome). Such methods are well known to those of ordinary skill in the art.

In some embodiments, the formulations of the present invention are useful for parenteral administration (*e.g*., intravenous, subcutaneous, intramuscular, intramedullary, and intraperitoneal). Therapeutic co-administration of some contemplated anticancer agents (*e.g*., therapeutic polypeptides) can also be accomplished using gene therapy reagents and techniques.

The anti-Notch antibody is administered to a subject in combination with one or more conventional anticancer agents as described herein. In preferred embodiments of the present invention, pharmaceutically acceptable carriers are biologically inert. In preferred embodiments, the pharmaceutical compositions of the present invention are formulated using pharmaceutically acceptable carriers well known in the art in dosages suitable for oral administration. Such carriers enable the pharmaceutical compositions to be formulated as tablets, pills, capsules, dragees, liquids, gels, syrups, slurries, solutions, suspensions and the like, for respective oral or nasal ingestion by a subject.

Pharmaceutical preparations for oral use can be obtained by combining the active compounds with solid excipients, optionally grinding the resulting mixture, and processing the mixture into granules, after adding suitable auxiliaries, if desired, to obtain tablets or dragee cores. Suitable excipients are carbohydrate or protein fillers such as sugars, including lactose, sucrose, mannitol, or sorbitol; starch from corn, wheat, rice, potato, *etc.;* cellulose such as methyl cellulose, hydroxypropylmethyl-cellulose, or sodium carboxymethylcellulose; gums including arabic and tragacanth; and proteins such as gelatin and collagen, If desired, disintegrating or solubilizing agents may be added, such as the cross-linked polyvinyl pyrrolidone, agar, alginic acid or a salt thereof such as sodium alginate.

For injection, the pharmaceutical compositions of the invention may be formulated in aqueous solutions, preferably in physiologically compatible buffers such as Hanks' solution, Ringer's solution, or physiologically buffered saline. For tissue or cellular administration, penetrants appropriate to the particular barrier to be permeated are used. Such penetrants are known to those skilled in the art.

Pharmaceutical compositions suitable for use in the present invention include compositions wherein the active ingredients are contained in an effective amount to achieve the intended purpose. The determination of an effective amount of an agent is well within the skills of those in the pharmacological arts, especially in view of the disclosure provided herein.

In addition to the active ingredients, preferred pharmaceutical compositions may contain suitable pharmaceutically acceptable carriers comprising excipients and auxiliaries that facilitate processing of the active compounds into pharmaceutically useful forms.

The pharmaceutical compositions of the present invention may be manufactured using any acceptable techniques for preparing pharmaceutical compositions including, but not limited to, by means of conventional mixing, dissolving, granulating, dragee-making, levigating, emulsifying, encapsulating, entrapping or lyophilizing processes, and the like.

Ingestible formulations of the present compositions may further include any material approved by the United States Department of Agriculture for inclusion in foodstuffs and substances that are generally recognized as safe (GRAS), such as food additives, flavorings, colorings, vitamins, minerals, and phytonutrients. The term phytonutrients, as used herein, refers to organic compounds isolated from plants that have a biological effect, and includes, but is not limited to, compounds of the following classes: isoflavonoids, oligomeric proanthcyanidins, indol-3-carbinol, sulforaphone, fibrous ligands, plant phytosterols, ferulic acid, anthocyanocides, triterpenes, omega 3/6 fatty acids, polyacetylene, quinones, terpenes, cathechins, gallates, and quercitin.

Dragee cores are provided with suitable coatings such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, (*i.e.*, dosage).

Pharmaceutical preparations contemplated for oral administration include push-fit capsules made of gelatin, as well as soft sealed capsules of gelatin and a coating such as glycerol or sorbitol. Push-fit capsules can contain the active ingredients mixed with fillers or binders such as lactose or starches, lubricants such as talc or magnesium stearate, and, optionally, stabilizers. In some soft capsules , the active compounds are dissolved or suspended in a suitable liquid or solvent, such as fatty oils, liquid paraffin, or liquid polyethylene glycol, with or without stabilizers.

Pharmaceutical formulations for parenteral administration include aqueous solutions of the active compounds. Aqueous injection suspensions optionally contain substances that increase the viscosity of the suspension such as sodium carboxymethyl cellulose, sorbitol, or dextran. Additionally, suspensions of the active compounds may be prepared as appropriate oily injection suspensions. In this aspect, suitable lipophilic solvents or vehicles include fatty oils such as sesame oil, or synthetic fatty acid esters, such as ethyl oleate or triglycerides, or liposomes. Optionally, suspensions contain suitable stabilizers or agents that increase the solubility of the compounds thus allowing for the preparation of highly concentrated solutions.

Dosing and administration regimes are tailored by the clinician, or others skilled in the pharmacological arts, based upon well known pharmacological and therapeutic considerations including, but not limited to, the desired level of therapeutic effect, and the practical level of therapeutic effect obtainable. Generally, it is advisable to follow well-known pharmacological principles for administrating chemotherapeutic agents (e.g., it is generally advisable to not change dosages by more than 50% at time and no more than every 3-4 agent half-lives). For compositions that have relatively little or no dose-related toxicity considerations, and where maximum efficacy (e.g., destruction of cancer cells) is desired, doses in excess of the average required dose are not uncommon. This approach to dosing is commonly referred to as the "maximal dose" strategy.

Additional dosing considerations relate to calculating proper target levels for the agent being administered, the agent's accumulation and potential toxicity, stimulation of resistance, lack of efficacy, and describing the range of the agent's therapeutic index.

Routine methods of titrating the agent's administration may be used. One common strategy for the administration is to set a reasonable target level for the agent in the subject. Agent levels may be measured in the subject's plasma. Proper dose levels and frequencies are then designed to achieve the desired steady-state target level for the agent. Actual, or average, levels of the agent in the subject are monitored (*e.g*., hourly, daily, weekly, *etc*.) such that the dosing levels or frequencies can be adjusted to maintain target levels. Of course, the pharmacokinetics and pharmacodynamics (*e.g.*, bioavailability, clearance or bioaccumulation, biodistribution, drug interactions, *etc*.) of the particular agent or agents being administered can potentially impact what are considered reasonable target levels and thus impact dosing levels or frequencies.

Target-level dosing methods typically rely upon establishing a reasonable therapeutic objective defined in terms of a desirable range (or therapeutic range) for the agent in the subject. In general, the lower limit of the therapeutic range is roughly equal to the concentration of the agent that provides about 50% of the maximum possible therapeutic effect. The upper limit of the therapeutic range is usually established by the agent's toxicity and not by its efficacy. The upper limit of the therapeutic range for a particular agent may be the concentration at which less than 5 or 10% of subjects exhibit toxic side effects. In some embodiments, the upper limit of the therapeutic range is about two times, or less, than the lower limit. Those skilled in the art will understand that these dosing consideration are highly variable and to some extent individualistic (e.g., based on genetic predispositions, immunological considerations, tolerances, resistances, and the like). Thus, effective target dosing levels for an agent in a particular subject may be 1, ... 5, ... 10, .. . 15, ... 20, ... 50, ... 75, ... 100, ... 200, ... X%, greater than optimal in another subject. Conversely, some subjects may suffer significant side effects and toxicity related health issues at dosing levels or frequencies far less (1, ... 5, ... 10, ... 15, ... 20, ... 50, ... 75, ... 100, ... 200, ... X%) than those typically producing optimal therapeutic levels in some or a majority of subjects. In the absence of more specific information, target administration levels are often set in the middle of the therapeutic range.

Disclosed herein are intermittent dosing methods, since marked fluctuations in agent concentration between doses are generally undesirable. In situations where the absorption and distribution of the agent are balanced and spontaneous, concentration fluctuations are dictated by the agent's elimination half-life.

In situations where the administered compositions are relatively nontoxic, maximal dosing methods can be used, because even concentrations of the agent several times that necessary for ensuring therapeutic efficacy are well tolerated. In these cases , the dosing intervals are lengthened such that the concentration of the agent in the subject's system remains within the range of therapeutic effectiveness for relatively long periods of time before being cleared from the subject and additional administrations are required to bring the agent's level back into the therapeutically effective range. Thus, dosing intervals may be longer than the agent's elimination half life.

In other situations, where the compositions have relatively narrow therapeutic ranges, it may be important calculate the maximum and minimum concentrations that will occur at particular dosing interval(s). The minimal steady-state concentration of administered agents may be determined using equations, optionally corrected for the bioavailability of the agents, which are well known to those skilled in the pharmacological arts.

Where the agents follow multiexponential kinetics and the agents are administered orally, the estimation of the maximal steady-state concentration involves manipulation of several exponential constants concerning agent distribution and absorption.

Also described herein are methods for administering loading doses of an agent, or agents, to a subject. As used herein, a "loading dose" is one or a series of doses that when given at the onset of a treatment quickly provide the target concentration of the therapeutic agent. Loading doses may be administered to a subject having an immediate need for the target level of an agent in relation to the time required to attain a steady-state target level of the agent provided using a constant rate of administration. Various negative considerations should be weighed against the exigency of the subject's condition and her need for a loading dose prior to its administration. These considerations include, but are not limited to: 1) loading doses are often administered in one large bolus which may abruptly subject the patient to a toxic concentration of the agent; 2) agents with long half-lives will remain at levels above the target-level as compared to agents administered under lower constant rate schemes. Loading doses are often large, rapid, and given parenterally, thus dangerous side effects can potentially occur at the site of administration before the agent can obtain equilibrium in the subject's plasma.

The clinician may rationally design an individualized dosing regimen based on known pharmacological principles and equations. In general, the clinician designs an individualized dosing regimen based on knowledge of various pharmacological and pharmacokinetic properties of the agent, including, but not limited to, F (fractional bioavailability of the dose), Cp (concentration in the plasma), CL (clearance/clearance rate), Vss (volume of drug distribution at steady state) Css (concentration at steady state), and tl/2 (drug half-life), as well as information about the agent's rate of absorption and distribution. Those skilled in the art are referred to any number of well known pharmacological texts (*e.g*., Goodman and Gilman's, Pharmaceutical Basis of Therapeutics, 10th ed., Hardman et al., eds., 2001) for further explanation of these variables and for complete equations illustrating the calculation of individualized dosing regimes. Those skilled in the art also will be able to anticipate potential fluctuations in these variables in individual subjects. For example, the standard deviation in the values observed for F, CL, and Vss is typically about 20%, 50%, and 30%, respectively. The practical effect of potentially widely varying parameters in individual subjects is that 95% of the time the Css achieved in a subject is between 35 5 and 270% that of the target level. For drugs with low therapeutic indices, this is an undesirably wide range. Those skilled in the art will appreciate, however, that once the agent's Cp (concentration in the plasma) is measured, it is possible to estimate the values of F, CL, and Vss directly. This allows the clinician to effectively fine tune a particular subject's dosing regimen.

Continuing therapeutic drug monitoring techniques may be used to further adjust an individual's dosing methods and regimens. For example, Css data may be used is to further refine the estimates of CL/F and to subsequently adjust the individual's maintenance dosing to achieve desired agent target levels using known pharmacological principles and equations. Therapeutic drug monitoring can be conducted at practically any time during the dosing schedule. Monitoring may be carried out at multiple time points during dosing and especially when administering intermittent doses. For example, drug monitoring can be conducted concomitantly, within fractions of a second, seconds, minutes, hours, days, weeks, months, *etc.*, of administration of the agent regardless of the dosing methodology employed (*e.g.*, intermittent dosing, loading doses, maintenance dosing, random dosing, or any other dosing method). However, those skilled in the art will appreciate that when sampling rapidly follows agent administration the changes in agent effects and dynamics may not be readily observable because changes in plasma concentration of the agent may be delayed (*e.g*., due to a slow rate of distribution or other pharmacodynamic factors). Accordingly, subject samples obtained shortly after agent administration may have limited or decreased value.

The primary goal of collecting biological samples from the subject during the predicted steady-state target level of administration is to modify the individual's dosing regimen based upon subsequently calculating revised estimates of the agent's CL/F ratio. However, those skilled in the art will appreciate that early postabsorptive drug concentrations do not typically reflect agent clearance. Early postabsorptive drug concentrations are dictated principally by the agent's rate of absorption, the central, rather than the steady state, volume of agent distribution, and the rate of distribution. Each of these pharmacokinetic characteristics have limited value when calculating therapeutic long-term maintenance dosing regimens.

Accordingly, when the objective is therapeutic long-term maintenance dosing, biological samples are obtained from the subject, cells, or tissues of interest well after the previous dose has been administered, and even more preferably shortly before the next planned dose is administered.

Where the therapeutic agent is nearly completely cleared by the subject in the interval between doses, then biological samples from the subject may be collected at various time points following the previous administration, and most preferably shortly after the dose was administered.

When low clearance of the agent is problematic, and toxicity issues are likely to result from its accumulation, agent concentrations may be measured immediately before the administration of the subsequent dose. In these situations, the determination of maximal and minimal agent concentrations are preferred.

When a constant maintenance dosage is administered, steady state may be reached only after expiration of four agent half-lives. Samples collected too soon after dosing begins do not accurately reflect agent clearance. However, for potentially highly toxic agents, significant toxicity and damage may already have ensued before expiration of the agent's fourth half-life. Thus, in some instances when it is important to maintain control over agent concentrations, a first sample is taken after two half-lives, assuming a loading dose has not been administered. If agent concentration already exceeds 90% of the eventual expected mean steady-state concentration, the dosage rate is halved, and another sample obtained following an additional two half lives. The dosage is halved again if this sample once more exceeds the target level. If the first concentration does not exceed tolerable limits, subsequent administrations are given at the initial dose rate. If the concentration is lower than expected, the steady state can likely be achieved in about two half-lives, and at this point the dosage rate can be adjusted as described herein.

In intermittent dosages, an additional concern related to timing of collection of concentration information, is if the sample was obtained immediately before the next scheduled dose, concentration will be at a minimal value, not the mean; however, as discussed herein, the estimated mean concentration can be calculated using equations known in the pharmacological arts.

When administering therapeutic agents having first-order kinetics, the average, minimum, and maximum concentrations at steady state are linearly related to the dose and dosing rate. Thus, in these instances, the ratio between the measured and the desired agent concentrations is used to adjust dosing.

Computer programs are helpful in designing dosing regimens. Typically, these programs take into account the measured drug concentrations and various factors (*e.g*., measured or predicted) related to the individual subjects.

Biological samples can be acquired including, but not limited to, polynucleotides, polypeptides, lipids, carbohydrates, glycolipids, ionic species, metabolites, inorganic molecules, macromolecules and macromolecular precursors as well as cell fractions, blood (*e.g.,* cellular and soluble or insoluble blood components including, but not limited to, plasma, serum, metabolites, factors, enzymes, hormones, and organic or inorganic molecules), exudates, secretions, sputum, excreta, cell and tissue biopsies, CNS fluids (cerebrospinal fluid), secretions of lachrymal, salivary, and other glands, seminal fluids, *etc.*, and combinations of these or any other subcellular, cellular, tissular, organismal, systemic, or organismic biological materials. Biological samples taken from a subject can be analyzed for chemical or biochemical changes (*e.g.*, changes in gene expression) or other effects resultant from administration of the therapeutic agent. Further biological sample and sampling consideration are described below.

The biological and pharmacological effects of the therapeutic compositions may be determined using routine laboratory procedures on the collected samples including, but not limited to, microscopy (*e.g.*, light, fluorescence (confocal fluorescence, immunofluorescence), phase-contrast, differential interference-contrast, dark field, or electron (transmission, scanning, cryo-), NMR, autoradiography), cell sorting techniques (*e.g.*, fluorescence-activated), chromatography techniques (*e.g.,* gel-filtration, ion exchange, hydrophobic, affinity, HPLC), electrophoretic techniques (*e.g.,* SDS-PAGE, 2D-, 3D-, isoelectric focusing), ultracentrifugation, immunocytochemical and immunohistochemical technologies (*e.g*., ELISA, Western blotting, Immuno blotting), nucleic acid, including recombinant, technologies (*e.g*., PCR (inverse, reverse, nested), Northern blotting, Southern blotting, Southwestern blotting, *in situ* hybridization, FISH, nick-translation, DNAse footprinting, DNAse hypersensitivity site mapping, Maxam-Gilbert sequencing, Sanger sequencing, gel-shift (mobility shift) analysis, S 1 nuclease analysis, RNAse protection assay, CAT assays, transgenic techniques, knock-out techniques, and reporter gene systems), amino acid analysis (*e.g.*, Edman degradation), morphological, pathological, or phenotypical observations, and other observations with or without aid of instrumentation.

Subjects may be questioned directly or indirectly regarding their state of health and any changes attributable to the administration of the therapeutic compositions (*e.g.,* drugs, small molecules, and other therapeutic agents and techniques) and methods.

Various interpatient and intrapatient pharmacokinetic considerations affect the design of dosing and administration regimens for individual patients. For any given drug, there may be wide variations in its pharmacokinetic properties in a particular subject, and up to one-half or more of the total variation in eventual response. The importance of these variable factors depends in part upon the agent and its usual route of elimination. For example, agents that are primarily removed by the kidneys and excreted unchanged into the urinary system, tend to show less interpatient variability in subjects with similar renal function than agents that are metabolically inactivated. Agents that are extensively metabolized, and agents that have high metabolic clearance and large first-pass elimination rates show large differences in interpatient bioavailability. Agents with slower rates of biotransformation typically have the largest variation in elimination rates among individual subjects. Differences in subject genotypes also plays an important part in determining different metabolic rates. Pathological and physiological variations in individual subjects' organ functions (*e.g.,* renal or hepatic diseases) are major factors that can affect an agent's rate of disposition. Kidney or liver diseases often impair drug disposition and thus increase interpatient drug variability. Other factors (*e.g.*, age) can also affect the responsiveness of targeted cells and tissues (*e.g.*, the brain) to a particular composition or method and can alter the expected range of the therapeutic target level for the agent.

When invasive patient samples (*e.g*., blood, serum, plasma, tissues, *etc.*) are necessary to determine the concentration of the therapeutic agent(s) in a subject, design of the collection procedures should be undertaken after considering various criteria including, but not limited to: 1) whether a relationship exists between the concentration of the agent and any desired therapeutic effects or avoidable toxic effects; 2) whether these is substantial interpatient variability, but small intrapatient variation in agent disposition; 3) whether it is otherwise difficult or impractical to monitor the effects of the agent; and 4) whether the therapeutic concentration of the agent is close to the toxic concentration. Concentration measurements may be supplemented with additional measurements of pharmacokinetic, pharmacodynamic, or pharmacological effects.

In some instances, considerable interpatient response variations exist after the concentration of agent has been adjusted to the target level. For some agents, this pharmacodynamic variability accounts for much of the total variation in subject response. The relationship among the concentration of an agent and the magnitude of the observed response may be complex, even when responses are measured in simplified systems *in vitro,* although typically a sigmoidal concentration-effect curve is seen. Often there is no single characteristic relationship between agent concentration (*e.g.*, in the subject's plasma) and measured effect. The concentration-effect curve may be concave upward or concave downward. The data plots may be linear, sigmoid, or in an inverted U-shape. Moreover, the resulting concentration-effect relationship curves can be distorted if the response being measured is a composite of several effects. The composite concentration-effect curves may be resolved into simpler component curves using calculations and techniques available to those skilled in the art.

The simplified concentration-effect relationships, regardless of their exact shape, can be viewed as having four characteristic variables: potency, slope, maximal efficacy, and individual variation. Those skilled in the art will appreciate that the potency of an agent is measured by the intersection of the concentration-effect curve with the concentration axis. Although potency is often expressed as the dose of an agent required to produce the desired effect, it is more appropriately expressed as relating to the concentration of the agent in the subject (*e.g*., in plasma) that most closely approximates the desired situation in an *in vitro* system to avoid complicating pharmacokinetic variables. Although potency affects agent dosing, knowledge of an agent's potency alone is relatively unimportant in clinical use so long as a dose sufficient to obtain the target level can be conveniently administered to the subject. It is generally accepted that more potent agents are not necessarily therapeutically superior to less potent agents. One exception to this principle, however, is in the field of transdermal agents.

The maximum effect that an agent can induce in a subject is called its maximal or clinical efficacy. An agent's maximal efficacy is typically determined by the properties of the agent and its receptor-effector system and is reflected in the plateau of the concentration-effect curve. In clinical use, however, an agent's dosage may be limited by undesirable effects (*e.g*., toxicity), and the true maximal efficacy of the agent may not be practically achievable without harming the subject.

The slope and shape of the concentration-effect curve reflects the agent's mechanism of action, including the shape of the curve that, at least in part, describes binding to the agent's receptor. The rise of the concentration-effect curve indicates the clinically useful dosage range of the agent. Those skilled in the art will appreciate that the dosage ranges recited herein are approximations based on sound pharmacological principles and that actual responses will vary among different individuals given the same concentration of an agent, and will even vary in particular individuals over time. It is well known that concentration-effect curves are either based on an average response, or are tailored to reflect an actual response in a particular individual at a particular time.

The concentration of an agent that produces a specified effect in a particular subject is called the individual effective concentration. Individual effective concentrations usually show a lognormal distribution, resulting in a normal variation curve from plotting the logarithms of the concentration against the frequency of achieving the desired effect. A cumulative frequency distribution of individuals achieving the desired effect as a function of agent concentration is called the concentration-percent curve or quantal concentration-effect curve. The shape of this curve is typically sigmoidal. The slope of the concentration-percent curve is an expression of the pharmacodynamic variability in the population rather than an expression of the concentration range from a threshold to a maximal effect in the individual patient.

Those skilled in the art will appreciate that the median effective dose (EDso) is the dose of an agent sufficient to produce the desired effect in 50% of the population.

In preclinical drug studies, the dose (MTD) is determined in experimental animals. The ratio of the MTD to the End.50 is an indication of the agent's therapeutic index and is a measurement of the selectivity of the agent in producing its desired effects. In clinical studies, the dose, or preferably the concentration, of an agent sufficient to produce toxic effects is compared to the concentration required for the therapeutic effects in the population to provide a clinical therapeutic index. However, due to individual pharmacodynamic variations in the population, the concentration or dose of an agent required to produce the therapeutic effect in most subjects occasionally overlaps the concentration that produces toxicity in some subjects despite the agent having a large therapeutic index. Those skilled in the art will appreciate that few therapeutic agents produce a single effect, thus, depending on the effect being measured, the therapeutic index for the agent may vary.

Described herein are approaches to individualize dosing levels and regimens. Optimal treatment regimens for particular subjects are designed after considering a variety of biological and pharmacological factors including, but not limited to, potential sources of variation in subject response to the administered agent(s), diagnosis specifics (e.g., severity and stage of disease, presence of concurrent diseases, etc.), other prescription and non prescription medications being taken, predefined efficacy goals, acceptable toxicity limits, cost-benefit analyses of treatment versus non treatment or treatment with other various available agents, likelihood of subject compliance, possible medication errors, rate and extent of agent absorption, the subject's body size and compositions, the agent's distribution, the agent's pharmacokinetic profile (*e.g*., physiological variables, pathological variables, genetic factors and predispositions, drug interactions, potential drug resistances, predicted rate of clearance), potential drug-receptor interactions, functional state, and placebo effects.

The clinician may select an appropriate marker for measuring the ultimate effectiveness of the administered agent(s) in the subject. Appropriate markers of an agent's effectiveness include a decrease (or increase) in some measurable biological state, condition, or chemical level (*e.g*., toxin load, viral titer, antigen load, temperature, inflammation, blood cell counts, antibodies, tumor morphology, and the like). A large number of diagnostic procedures and tests are available for gathering information on various markers including, but not limited to, cell culture assays (*e.g*., invasion assays in soft-agar and the like), radiographic examination (*e.g.*, chest X-ray), computed tomography, computerized tomography, or computerized axial tomography (CAT) scans, positron emission tomography (PET) scans, magnetic resonance imaging (MRI or NMRI], mammography, ultrasonography (transvaginal, transcolorectal), scintimammography (*e.g.,* technetium 99m sestamibi, technetium-99m tetrofosmin), aspiration (*e.g*., endometrial), palpation, PAP tests (*e.g*., smears), sigmoidoscopy (*e.g.*, flexible fiberoptic), fecal occult blood testing (*e.g*., Guaiac-based FOBT), digital rectal examination, colonoscopy, virtual colonoscopy (also known as colonography), barium enema, stool analysis (*See e.g.,* K.W. Kinzler and B. Vogelstein, Cell, 87(2):159-70 (1996); S.M. Dong et al., J. Natl. Cancer Inst., 93(11):858-865 (2001); G. Traverso et al., N. Engl. J. Med., 346(5):311-20 (2002), G. Traverso et al., Lancet, 359(9304):403 (2002); and D.A. Ahlquist et al., Gastroenterology, 119(5):1219-1227, (2000)), serum prostate-specific antigen (PSA) screening, endoscopy, gallium scans, marrow and tissue biopsies *(e.g.,* core-needle, percutaneous needle biopsy, thoracotomy, endometrial, *etc*.) and histological examinations, direct and/or indirect clinical observations (*e.g.*, patient surveys, inquiries, or questionnaires), cytological sampling and collection of biological tissues, fluids, and markers therein, (*e.g.,* blood, urine *(e.g.,* hematuria screening, urinary cytologic examinations), sputum *(e.g.,* sputum cytology), feces, CNS fluids *(e.g.,* LPs, spinal taps), blood products, including proteins and peptides (*e.g*., Bcl-2 family proteins), cancer markers (*e.g.,* CA 15-3 (breast cancer), CEA , breast and pancreas cancers), p53 gene product, MIC2 gene product), metabolites (*e.g*., vanillylmandelic acid (VMA), and homovanillic acid (HVA)), antigens (*e.g.*, serum alpha-fetoprotein (AFP)), salts, minerals, vitamins, soluble factors, insoluble factors, nucleic acids, and the like).

The toxicity and therapeutic efficacy of agents may be determined using standard pharmaceutical procedures in cell cultures or experimental animals, *e.g.*, for determining the MTD and the ED₅₀. Agents that exhibit large therapeutic indices are preferred. The data obtained from cell culture assays or animal models can be used to formulate dosing ranges in, for example, mammals (*e.g*., humans, *Equus caballus, Felis catus,* and *Canis familiaris, etc.).* Preferable dosing concentrations are near the calculated or observed ED₅₀ value for an agent. More preferable dosing concentrations are near an agent's ED₅₀ value and cause little or no toxicity. Any given dosage may vary within, exceed, or be less than, the therapeutic index for any particular agent, depending upon the formulation, sensitivity of the patient, and the route of administration.

### EXPERIMENTAL DATA

### Methods

**Tumor establishment and analysis.** Human breast cancer tumors were established in 8-week old female NOD-SCID mice as previously reported¹,

**Cell culture**. Passage-1 or passage-2 primary breast cancer cells were plated in triplicate 12-collagen-coated well dishes in HAM-F12 medium supplemented with Fetal Bovine Serum (1%), Insulin (5µg/ml), Hydrocortisone (1µg/ml), EGF (10µg/ml), Choleratoxin (0.1µg/ml), Transferrin and Selenium (GIBCO BRL, recommended dilutions), pen/strep, and fungizone (GibcoBRL). Culture medium was replaced once every two days.

**Flow Cytometry.** To prepare cells for flow-cytometric analysis, single cell suspensions of human breast cancer tumors passaged once or twice in mice¹ were made by mincing tumors and digesting them with 200 u/ml of collagenase 3 (Worthington) in NI199 medium (Gibco/BRL, Rockville, MD) for 2-4 hours at 37° C with constant agitation. Antibodies included anti-CD44, anti-CD24 , anti-ESA-FITC (Biomeda, CA), anti-H2K, and goat-anti-human Notch4 (Santa Cruz Products, Santa Cruz, CA). Unless noted, antibodies were purchased from Pharmingen (San Diego, CA). Antibodies were directly conjugated to various fluorochromes depending on the experiment. In all experiments, mouse cells were eliminated by discarding H2K⁺ (class I MHC) cells during flow cytometry. Dead cells were eliminated using the viability dye 7-AAD. Flow cytometry was performed on a FACSVantage (Becton Dickinson, San Jose, CA).

Notch reporter assay. The HES-1 - Luciferase reporter construct was made using the HES-1 Notch response element³⁸. The fragment of the HES-1 murine gene between-194 and +160 was amplified by PCR and subcloned into a pGL2 basic vector (Promega) between the Kpnl and Bgl II sites. MCF-7 cells were co-transfected with the HES-1-luc construct and pSV2Neo and selected in medium containing geneticin. The transfected MCF-7 cells were cocultivated in 12 well plates in the presence and absence of the Notch4 polyclonal antibody (Santa Cruz; 20ug/ml final concentration), soluble Delta-Fc (13) or the Notch4 antibody blocking peptide (4mg/100ml final concentration, Santa Cruz Products). Luciferase assays were performed as described³⁸. Delta-Fc or Fc control proteins were concentrated from the supernatant of 293 cells that were engineered to secrete them¹¹, Delta-Fc or Fc control proteins were added to breast cancer cell cultures along with a cross-linking anti-Fc antibody (Jackson Immunoresearchl)¹¹.

**Apoptosis assays.** 10,000-20,000 tumorigenic T1 cells (ESA⁺CD44⁺CD24^{-/low}Lineage) or Lineage' tumor cells from T5, T7 and T8 were sorted by flow cytometry and grown on collagen-coated tissue culture plates. Anti-Notch4 polyclonal antibody (Santa Cruz , CA) was then added to the medium (20mg/ml final concentration) while PBS was added to the control plates. To block the anti-Notch4 antibody, the anti-Notch4 antibody was pre-incubated with the blocking peptide (Santa Cruz, CA) on ice for 30 minutes after which it was added to the medium. After 48 hrs, cells were trypsinized and collected. 10⁵ cells were suspended in HBSS 2% heat inactivated calf serum and then assayed for apoptosis using FAM-DEVD-FMK, a caroxyfluorescein-labeled peptide substrate specific to caspases 3 and 7 (CaspaTag™ Caspase Activity Kit, Intergen Company, NY) to detect active caspases in living cells. Caspase positive cells were distinguished from the negative ones using FACSVantage flow cytometer (Becton Dickinson, CA). PI staining for cell cycle and apoptosis was performed as described³⁹.

### Recombinant Adenovirus

The E3-deleted adenoviral vector designated Ad-GFP-dnMAML1was constructed as follows: The coding region of amino acids 1-302 of the Human MAML1 was generated from normal breast cells by PCR with a forward primer with an EcoRI site and an ATG start codon, and the reverse primer with an HpaI site. The resulting DNA fragment was cloned in the MSCVneoEB vector upstream of the IRES-GFP gene. The dnMAML1-IRES-GFP was then cloned into the adenovirus shuttle vector pACCMV2 at the EcoRI and BamHI sites. The recombinant adenovirus was then generated at the University of Michigan viral core. Cells were cultured in 12-well plates and infection events were carried out in triplicates. Viral infections were carried out by adding viral particles at various concentrations to culture medium without FBS. Initially, optimal viral concentration was determined for each cell line or primary cell by using AD-GFP to achieve an optimal balance of high gene expression and low viral titer to minimize cytotoxicity. After 4 hrs of incubation, the infection medium was supplemented with appropriate FBS amount for each cell type. Transduction efficiency (24 hours post-infection) was calculated by dividing the number of GFP positive cells by the total number of cells from three separate microscopic fields at a magnification of 40X or by Cellquest when FACS was used to examine GFP.

### PCR analysis

Gene expression analysis of the Fringes, Notches and their ligands was done by quantitative RT-PCR The primers for the PCR reactions for the genes examined were purchased from Applied Biosystems. 2000 tumorigenic and non-tumorigenic cells were isolated by flow cytometry in triplicate for each population and each assayed gene, using the markers described above. RNA isolation was done using Trizol following the GIBCO BRL protocol. cDNA generation was done using an OligodT anchor primer, and the PCR reactions were prepared using the Taqman Assay-on-demand system (Applied Biosystems). The PCR reactions were run on the ABI PRISM® 7900HT Sequence Detection System (AB), which is a high-throughput real-time PCR system that detects and quantifies nucleic acid sequences. Agarose gel electrophoresis analysis of the PCR products demonstrated a single PCR product (data not shown). Statistical analysis of differential gene expression was done using the 2^{ΔΔC}T method⁴⁰.

### Western Analysis:

Equal amounts of cell lysates from the, MCF-7 cells transfected with pcDNA3(MCF-7-V), MCF-7 cells transfected with a dominant negative FADD (MCF-7/ dnFADD)²⁷ , and 2 clones of MCF-7 cells transfected with Bel-X_{L} (MCF-7/BCL-X_{L}, clones 1 and 2)²⁸ were tested for the expression of the expected proteins. 293T cells were transiently transfected with pcDNA3-FLAG-BCL-X_{L}. The expression of FLAG-BCL-X_{L} protein hAD been verified using anti-FLAG antibody. To detect the AUI-tagged dnFADD protein anti-AUI mouse monoclonal Ab (1/1,000) was used (Covance, Berkeley-CA). BCL-X_{L} levels were detected using the anti-BCL-X s/l (S-18) rabbit polyclonal antibody (dilution 1/500) (Santa Cruz Biotecnology Inc., Santa Cruz-CA).

For Primary Tumors 1 Tumor 4 (designated T1 and T4), samples of 2000 cells from the tumorigenic ESA⁺CD44⁺CD24^{-low}Lineage⁻ population (designated T), or 2000 cells from the non-tumorigenic population (designated NT) from each of the tumors were sorted as described previously¹. For Primary tumors 2, 3 and 5 (designated T2, T3 and T5), 2000 H2K⁻ cells from each tumor were sorted as previously described. This represents a mixed population of tumorigenic and non-tumorigenic cancer cells. Each gene was analyzed using 3 independently sorted samples of cells. (+) indicates that expression of a gene was detected. (-) indicates that gene expression was not detected. (ND) indicates the-assay was not done. Analysis of each of the 4 Notch receptors is indicated, as well as the Notch ligands of the Delta and Jagged families. The Fringes (Lunatic, Manic and Radical) were also analyzed.

### Regulation of cancer stem cell proliferation through regulation of Notch pathway signaling

This section describes compounds and methods for regulating cell proliferation by targeting Notch pathway signaling without directly targeting Notch 4.

γ-*Secretase Inhibitor.* Cell viability was assayed by plating triplicate 20,000 MCF-7 cells that are shown to express *Notch* in 6-well plates. 48 hours later, 1.19µg/ml of y-secretase inhibitor was added to the cells. Total and Trypan Blue negative (viable) cells were counted 12, 24 and 48 hours later and % of viable cells compared to the control wells was determined.

Experiments conducted during the development of the present invention were designed to test the effect of *Notch* signaling blockage on the survival and proliferation of MCF-7 cells. Cells were co-cultured with a γ-secretase inhibitor, which inhibits the final cleavage of the *Notch* receptor, preventing the release of the *Notch* intra-cellular (IC) domain, essentially blocking *Notch* signaling (Karlstrom et al., J. Biol. Chem., 277:6763 (2002)). Cell viability was then assayed after exposure to the inhibitor. The percentage of viable cells was determined 12, 24 and 48 hours post-exposure. There was a significant decrease in viability after 24 hours, and most of the cells were dead after 48 hours. To determine the specificity of the γ-secretase inhibitor on the *Notch* signaling pathway, stably transfected MCF-7 cells were used with the reporter luciferase gene under the control of the *HES-1* promoter. The trans-activation or suppression of the *HES-1* promoter, a downstream element of *Notch* signaling, which then could be monitored with a standard luciferase assay. The cells were co-cultivated with the γ-secretase inhibitor and monitored the changes in luciferase activity. The inhibitor clearly decreased the activity of luciferase 12 hours after exposure while cell viability was still high, indicating that it specifically suppressed the expression of the *Notch* receptor.

To determine the mechanism by which inhibition of *Notch* signaling induced cell death in the MCF-7 cells, Caspase activation was determined in the cells upon exposure to the γ-secretase inhibitor. Twenty-four hours post-exposure, the accumulation of degraded DNA in the cells was assayed for, which is a characteristic of apoptosis. Compared to the control MCF-7 cell cultures, there was a substantial increase in the number of cells with activated caspase 3/7 in the ones exposed to the γ-secretase inhibitor. These data show that in the MCF-7 cell line, a blocked *Notch* signaling pathway induces apoptosis via Caspase 3/7 activation.

Experiments were conducted to test the effect of inhibition of *Notch* signaling on the ability of breast cancer cells from five different patients with breast tumors to proliferate *in vitro.* Cells from the patients were cultured. The role of *Notch* signaling was examined by exposing these 5 different cell cultures to the γ-secretase inhibitor. The cells were placed in a previously established cell culture medium on collagen-coated plates and allowed to grow. The inhibitor was then added to the cultures and a viability assay was conducted 48 hours later. The γ-secretase inhibitor had a dramatic effect on the viability of these cells as shown in Figure 7. These data demonstrated that *Notch* activation promoted the survival or proliferation of breast cancer cells in all 5 of these *de novo* tumors.

### Inhibition of Notch signaling in vivo inhibits tumor formation by tumorigenic cancer cells

Experiments were further conducted to determine whether the blocking of *Notch* signaling inhibited tumor formation by breast cancer cells *in vivo.* Previously, it was found that only a minority of the cancer cells in a breast cancer tumor, called tumorigenic cancer cells, form tumors in a xenograft mouse model while the majority of the cancer cells lack this capacity (Al-Hajj et al., Proc. Natl. Acad. Sci. USA 100:3983 (2003)). As few as 1000 tumorigenic cancer cells from patient breast tumor 1 (TP1) and 10,000 tumorigenic cancer cells from patient breast tumor 4 (TP4) are able to form new tumors in *NOD*/*SCID* mice (Al-Hajj et al., supra). Therefore, TP1 and TP4 tumorigenic cancer cells were incubated with either a control adenovirus or the dnMAML1 adenovirus for 3 hours. NOD/SCID mice were injected with 10,000 or 1,000 TP1 tumorigenic cancer cells of each group. Even though at the time of injection into the mice the vast majority of the cancer cells infected with either control or dnMAML1 adenoviruses are still viable, 0 out of 10 injections of cells infected with the dnMAML1 adenovirus infected TP1 cells formed tumors while all of the injections of cells infected with the control adenovirus did so. Similarly, all 5 injections of 10,000 TP4 cells infected with the control adenovirus formed tumors, while none of the injections of 10,000 cells infected with the dnMAML-1 adenovirus did. These data show that *Notch* signaling is important for cancer cells survival and the formation of new tumors *in vivo* and the blocking of this pathway leads to the loss of the ability of these cells to form tumors.

### REFERENCES

1. Al-Hajj, M., Wicha, M. S., Benito-Hernandez, A., Morrison, S. J. & Clarke, M. F. Prospective identification of tumorigenic breast cancer cells. Proc Natl Acad Sci USA 100, 3983-8 (2003).
2. Austin, J. & Kimble, J. glp-1 is required in the germ line for regulation of the decision between mitosis and meiosis in C. elegans. Cell 51, 589-99 (1987).
3. Berry, L., Westlund, B. & Schedl, T. Germ-line tumor formation caused by activation of glp-1, a Caenorhabditis elegans member of the Notch family of receptors. Development 124, 925-936 (1997).
4. Gallahan, D., Kozak, C. & Callahan, R. A new common integration region (int-3) for mouse mammary tumor virus on mouse chromosome 17. J Virol 61, 218-20 (1987).
5. Gallahan, D. & Callahan, R. The mouse mammary tumor associated gene INT3 is a unique member of the NOTCH gene family (NOTCH4). Oncogene 14, 1883-1890 (1997).
6. Lippman, M. E. High-dose chemotherapy plus autologous bone marrow transplantation for metastatic breast cancer. N Engl J Med 342, 1119-20 (2000).
7. Lapidot, T. et al. A cell initiating human acute myeloid leukaemia after transplantation into SCID mice. Nature 17, 645-648 (1994).
8. Park, I. K. et al. Bmi-1 is required for maintenance of adult self-renewing haematopoietic stem cells. Nature 423, 302-5 (2003).
9. Reya, T., Morrison, S. J., Clarke, M. F: & Weissman, I. L. Stem cells, cancer, and cancer stem cells. Nature 414, 105-11 (2001).
10. Reya, T. et al. A role for Wnt signalling in self-renewal of haematopoietic stem cells. Nature 423, 409-14 (2003).
11. Morrison, S. et al. Transient Notch activation initiates an irreversible switch from neurogenesis to gliogenesis by neural crest stem cells. Cell 101, 499-510 (2000).
12. Varnum-Finney, B. et all. Pluripotent, cytokine-dependent, hematopoietic stem cells are immortalized by constitutive Notch 1 signaling. Nat Med 6, 1278-81 (2000).
13. Artavanis-Tsakonas, S., Rand, M. D. & Lake, R. J. Notch signaling: cell fate control and signal integration in development. Science 284, 770-776 (1999).
14. Allman, D., Aster, J. C. & Pear, W. S. Notch signaling in hematopoiesis and early lymphocyte development. Immunol Rev 187, 75-86 (2002).
15. Henrique, D. et al. Maintenance of neuroepithelial progenitor cells by Delta-Notch signalling in the embryonic chick retina. Curr Biol 7, 661-70 (1997).
16. Christensen, S., Kodoyianni, V., Bosenberg, M., Friedman, L. & Kimble, J. tag-1, a gene required for lin-12 and glp-1 signaling in Caenorhabditis elegans, is homologous to human CBF1 and Drosophila Su(H). Development 122, 1373-83 (1996).
17. Weizen, S. et al. Activation of Notch-1 signaling maintains the neoplasitc phenotype in human Ras-transformed cells. Nature Medicine 8, 979-986 (2002).
18. Callahan, R. & Raafat, A. Notch signaling in mammary gland tumorigenesis. J Mammary.Gland.Biol.Neoplasia. 6, 23-36 (2001).
19. Brennan, K. & Brown, A. M. Is there a role for Notch signalling in human breast cancer? Breast Cancer Res 5, 69-75 (2003).
20. Imatani, A. & Callahan, R. Identification of a novel NOTCH-4/INT-3 RNA species encoding an activated gene product in certain human tumor cell lines. Oncogene 19, 223-231 (2000).
21. Nam, Y., Weng, A. P., Aster, J. C. & Blacklow, S. C. Structural requirements for assembly of the CSL.intracellular Notchl.Mastermind-like 1 transcriptional activation complex. J Biol Chem 278, 21232-9 (2003).
22. Petcherski, A. G. & Kimble, J. Mastermind is a putative activator for Notch. Curr Biol 10, R471-3 (2000).
23. Wu, L., Sun, T., Kobayashi, K., Gao, P. & Griffin, J. D. Identification of a family of mastermind-like transcriptional coactivators for mammalian notch receptors. Mol Cell Biol 22, 7688-700 (2002).
24. Jeffries, S., Robbins, D. J. & Capobianco, A. J. Characterization of a high-molecular-weight Notch complex in the nucleus of Notch(ic)-transformed RKE cells and in a human T-cell leukemia cell line. Mol Cell Biol 22, 3927-41 (2002).
25. Jehn, B. M., Bielke, W., Pear, W. S. & Osborne, B. A. Cutting edge: protective effects of notch-1 on TCR-induced apoptosis. J Immunol. 162, 635-638 (1999).
26. Deftos, M. L., He, Y. W., Ojala, E. W. & Bevan, M. J. Correlating notch signaling with thymocyte maturation. Immunity, 9, 777-786 (1998).
27. Chinnaiyan, A. M., O'Rourke, K., Tewari, M. & Dixit, V. M. FADD, a novel death domain-containing protein, interacts with the death domain of Fas and initiates apoptosis. Cell 81, 505-12 (1995).
28. Jaattela, M., Benedict, M., Tewari, M., Shayman, J. A. & Dixit, V. M. Bcl-x and Bcl-2 inhibit TNF and Fas-induced apoptosis and activation of phospholipase A2 in breast carcinoma cells. Oncogene 10, 2297-305 (1995).
29. Bruckner, K., Perez, L., Clausen, H. & Cohen, S. Glycosyltransferase activity of Fringe modulates Notch-Delta interactions. Nature 406, 411-415 (2000).
30. Jones, P. et al. Stromal expression of Jagged 1 promotes colony formation by fetal hematopoietic progenitor cells. Blood 92, 1505-11 (1998).
31. Shimizu, K. et al. Manic fringe and lunatic fringe modify different sites of the Notch2 extracellular region, resulting in different signaling modulation. J Biol.Chem. 276, 25753-25758 (2001).
32. Hicks, C. et al. Fringe differentially modulates Jagged1 and Delta1 signalling through Notch1 and Notch2. Nat. Cell Biol. 2, 515-520 (2000).
33. May, W. A. et al. EWS/FLI1-induced manic fringe renders NIH 3T3 cells tumorigenic. Nat Genet 17, 495-7 (1997).
34. Akashi, K. et al. Transcriptional accessibility for genes of multiple tissues and hematopoietic lineages is hierarchically controlled during early hematopoiesis. Blood 101, 383-9 (2003).
35. Lessard, J. & Sauvageau, G. Bmi-1 Determines the Proliferative Activity of Normal and Leukemic Stem Cells. Nature (2003).
36. Dick, J. E. Breast cancer stem cells revealed. Proc Natl Acad Sci U S A 100, 3547-9 (2003).
37. Hanahan, D. & Weinberg, R. A. The hallmarks of cancer. Cell 100, 57-70 (2000).
38. Korinek, V. et al. Two members of the Tcf family implicated in Wnt/beta-catenin signaling during embryogenesis in the mouse. Mol Cell Biol 18, 1248-56 (1998).
39. Clarke, M. F. et al. A recombinant bcl-x s adenovirus selectively induces apoptosis in cancer cells but not in normal bone marrow cells. Proc.Natl.Acad.Sci.U.S.A 92, 11024-11028 (1995).
40. Livak, K. J. & Schmittgen, T. D. Analysis of relative gene expression data using real-time quantitative PCR and the 2(-Delta Delta C(T)) Method. Methods 25, 402-8 (2001).

## Claims

1. An inhibitor of Notch signalling for use in a method of inhibiting tumorigenic cells to treat pancreatic cancer or breast cancer in a subject, wherein the inhibitor is an anti-Notch antibody that inhibits proliferation of tumorigenic cells, and wherein the method comprises co-administering the inhibitor and an anticancer agent to the subject, wherein the anticancer agent is a chemotherapeutic agent.

2. An inhibitor for use according to claim 1, wherein the antibody is a humanized or human antibody.

3. An inhibitor for use according to claim 1 or claim 2, wherein the cancer is breast cancer.

4. An inhibitor for use according to claim 3, wherein the anticancer agent is an antimetabolite.

5. An inhibitor for use according to claim 4, wherein the antimetabolite is a pyrimidine antagonist.

6. An inhibitor for use according to claim 5, wherein the pyrimidine antagonist is gemcitabine.

7. An inhibitor for use according to claim 3, wherein the anticancer agent is an alkaloid.

8. An inhibitor for use according to claim 7, wherein the alkaloid is a microtubule stabilizer.

9. An inhibitor for use according to claim 8, wherein the microtubule stabilizer is paclitaxel.

10. Use of an inhibitor of Notch signalling, wherein the inhibitor is an anti-Notch antibody that inhibits proliferation of tumorigenic cells, for the manufacture of a medicament for inhibiting tumorigenic cells to treat pancreatic cancer or breast cancer in a subject wherein the treatment comprises co-administeririg the inhibitor and an anticancer agent to the subject, wherein the anticancer agent is a chemotherapeutic agent.

11. Use of
(i) an inhibitor of Notch signalling, wherein the inhibitor is an anti-Notch antibody that inhibits proliferation of tumorigenic cells,
and
(ii) an anticancer agent, wherein the anticancer agent is a chemotherapeutic agent,
for the manufacture of a medicament for inhibiting tumorigenic cells to treat pancreatic cancer or breast cancer in a subject, wherein the treatment comprises co-administering the inhibitor and the anticancer agent to the subject.

12. Use according to claim 10 or 11, wherein the antibody is a humanized or human antibody.

13. Use according to any of claims 10 to 12, wherein the cancer is breast cancer.

14. Use according to claim 13, wherein the anticancer agent is an antimetabolite.

15. Use according to claim 14, wherein the antimetabolite is a pyrimidine antagonist.

16. Use according to claim 15, wherein the pyrimidine antagonist is gemcitabine.

17. Use according to claim 13, wherein the anticancer agent is an alkaloid.

18. Use according to claim 17, wherein the alkaloid is a microtubule stabilizer.

19. Use according to claim 18, wherein the microtubule stabilizer is paclitaxel.

## Patentansprüche

1. Inhibitor der Notch-Signalgebung zur Verwendung in einem Verfahren zum Hemmen tumorbildender Zellen zum Behandeln von Bauchspeicheldrüsenkrebs oder Brustkrebs bei einem Individuum, worin der Inhibitor ein Anti-Notch-Antikörper ist, der die Proliferation von tumorbildenden Zellen hemmt, und worin das Verfahren das gemeinsame Verabreichen des Inhibitors und eines Mittels gegen Krebs an das Individuum umfasst, worin das Mittel gegen Krebs ein Chemotherapeutikum ist.

2. Inhibitor zur Verwendung nach Anspruch 1, worin der Antikörper ein humanisierter oder menschlicher Antikörper ist.

3. Inhibitor zur Verwendung nach Anspruch 1 oder Anspruch 2, worin der Krebs Brustkrebs ist.

4. Inhibitor zur Verwendung nach Anspruch 3, worin das Mittel gegen Krebs ein Antimetabolit ist.

5. Inhibitor zur Verwendung nach Anspruch 4, worin der Antimetabolit ein Pyrimidinantagonist ist.

6. Inhibitor zur Verwendung nach Anspruch 5, worin der Pyrimidinantagonist Gemischcitabin ist.

7. Inhibitor zur Verwendung nach Anspruch 3, worin das Mittel gegen Krebs ein Alkaloid ist.

8. Inhibitor zur Verwendung nach Anspruch 7, worin das Alkaloid ein Mikrotubuli-Stabilisator ist.

9. Inhibitor zur Verwendung nach Anspruch 8, worin der Mikrotubuli-Stabilisator Paclitaxel ist.

10. Verwendung eines Inhibitors der Notch-Signalgebung, worin der Inhibitor ein Anti-Notch-Antikörper ist, der die Proliferation von tumorbildenden Zellen hemmt, zur Herstellung eines Medikaments zur Hemmung von tumorbildenden Zellen, um Bauchspeicheldrüsenkrebs oder Brustkrebs bei einem Individuum zu behandeln, worin die Behandlung das gemeinsame Verabreichen des Inhibitors und eines Mittels gegen Krebs an das Individuum umfasst, worin das Mittel gegen Krebs ein Chemotherapeutikum ist.

11. Verwendung von
(i) einem Inhibitor der Notch-Signalgebung, worin der Inhibitor ein Anti-Notch-Antikörper ist, der die Proliferation von tumorbildenden Zellen hemmt, und
(ii) einem Mittel gegen Krebs, worin das Mittel gegen Krebs ein Chemotherapeutikum ist,
zur Herstellung eines Medikaments zur Hemmung von tumorbildenden Zellen, um Bauchspeicheldrüsenkrebs oder Brustkrebs bei einem Individuum zu behandeln, worin die Behandlung das gemeinsame Verabreichen des Inhibitors und des Mittels gegen Krebs an das Individuum umfasst.

12. Verwendung nach Anspruch 10 oder 11, worin der Antikörper ein humanisierter oder menschlicher Antikörper ist.

13. Verwendung nach einem der Ansprüche 10 bis 12, worin der Krebs Brustkrebs ist.

14. Verwendung nach Anspruch 13, worin das Mittel gegen Krebs ein Antimetabolit ist.

15. Verwendung nach Anspruch 14, worin der Antimetabolit ein Pyrimidinantagonist ist.

16. Verwendung nach Anspruch 15, worin der Pyrimidinantagonist Gemcitabin ist.

17. Verwendung nach Anspruch 13, worin das Mittel gegen Krebs ein Alkaloid ist.

18. Verwendung nach Anspruch 17, worin das Alkaloid ein Mikrotubuli-Stabilisator ist.

19. Verwendung nach Anspruch 18, worin der Mikrotubuli-Stabilisator Paclitaxel ist.

## Revendications

1. Inhibiteur des signaux Notch pour utilisation dans une méthode d'inhibition de cellules oncogènes pour traiter le cancer du pancréas ou le cancer du sein chez un sujet, où l'inhibiteur est un anticorps anti-Notch qui inhibe la prolifération des cellules oncogènes, et où la méthode comprend la coadministration de l'inhibiteur et d'un agent anticancer ou sujet, où l'agent anticancer est un agent chimiothérapeutique.

2. Inhibiteur pour utilisation selon la revendication 1, où l'anticorps est un anticorps humanisé ou humain.

3. Inhibiteur pour utilisation selon la revendication 1 ou la revendication 2, où le cancer est le cancer du sein.

4. Inhibiteur pour utilisation selon la revendication 3, où l'agent anticancer est un anti-métabolite.

5. Inhibiteur pour utilisation selon la revendication 4, où l'antimétabolite est un antagoniste de la pyrimidine.

6. Inhibiteur pour utilisation selon la revendication 5, où l'antagoniste de la pyrimidine est la gemcitabine.

7. Inhibiteur pour utilisation selon la revendication 3, où l'agent anticancer est un alcaloïde.

8. Inhibiteur pour utilisation selon la revendication 7, où l'alcaloïde est un stabilisateur de microtubules.

9. Inhibiteur pour utilisation selon la revendication 8, où le stabilisateur de microtubules est le paclitaxel.

10. Utilisation d'un inhibiteur des signaux de Notch, où l'inhibiteur est un anticorps anti-Notch qui inhibe la prolifération des cellules oncogènes, pour la fabrication d'un médicament pour inhiber les cellules oncogènes pour traiter le cancer du pancréas ou le cancer du sein chez un sujet, où le traitement comprend la co-administration de l'inhibiteur et d'un agent anticancer au sujet, où l'agent anticancer est un agent chimiothérapeutique.

11. Utilisation de
(i) un inhibiteur des signaux de Notch, où l'inhibiteur est un anticorps anti-Notch qui inhibe la prolifération des cellules oncogènes, et
(ii) un agent anticancer, où l'agent anticancer est un agent chimiothérapeutique,
pour la fabrication d'un médicament pour inhiber les cellules oncogènes pour traiter le cancer du pancréas ou le cancer du sein chez un sujet, où le traitement comprend la coadministration de l'inhibiteur et de l'agent anticancer au sujet.

12. Utilisation selon la revendication 10 ou 11, où l'anticorps est un anticorps humanisé ou humain.

13. Utilisation selon l'une quelconque des revendications 10 à 12, où le cancer est le cancer du sein.

14. Utilisation selon la revendication 13, où l'agent anticancer est un anti-métabolite.

15. Utilisation selon la revendication 14, où l'anti-métabolite est un antagoniste de la pyrimidine.

16. Utilisation selon la revendication 15, où l'antagoniste de la pyrimidine est la gemcitabine.

17. Utilisation selon la revendication 13, où l'agent anticancer est un alcaloïde.

18. Utilisation selon la revendication 17, où l'alcaloïde est un stabilisateur de microtubules.

19. Utilisation selon la revendication 18, où le stabilisateur de microtubules est le paclitaxel.
